# EUROPEAN PATENT SPECIFICATION

(11) **EP 2 164 503 B1**
(45) Date of publication and mention of the grant of the patent: **18.04.2012**
(21) Application number: 08767926.2
(22) Date of filing: 29.05.2008
(51) Int. Cl.: C12N 5/077

(54) **BONE AUGMENTATION UTILIZING MUSCLE-DERIVED PROGENITOR COMPOSITIONS, AND TREATMENTS THEREOF**
KNOCHENAUFBAU ANHAND VON VORLÄUFERZUSAMMENSETZUNGEN AUS MUSKELN UND BEHANDLUNGEN DAMIT
AUGMENTATION OSSEUSE OBTENUE À L'AIDE DE COMPOSITIONS DE CELLULES PROGÉNITRICES DÉRIVÉES DES MUSCLES ET TRAITEMENTS CORRESPONDANTS

(30) Priority: 29.05.2007 US 940576 P; 14.09.2007 US 972476 P
(43) Date of publication of application: 24.03.2010
(73) Proprietor: University of Pittsburgh - Of the Commonwealth System of Higher Education, Pittsburgh, PA 15260 (US)
(72) Inventor: PAYNE, Thomas, Pittsburgh, PA 15260 (US); PRUCHNIC, Ryan, Pittsburgh, PA 15260 (US); JANKOWSKI, Ronald, Pittsburgh, PA 15260 (US)
(74) Representative: Crump, Julian Richard John
(86) International application number: PCT/US2008/006781
(87) International publication number: WO 2008/153813

(56) References cited:
- WO-A1-2008/076435
- WO-A2-01/19966
- US-A1- 2005 265 978
- US-B1- 6 866 842
- US-B1- 7 115 417
- SUN J-S ET AL: "The role of muscle-derived stem cells in bone tissue engineering" BIOMATERIALS, ELSEVIER SCIENCE PUBLISHERS BV., BARKING, GB LNKD- DOI:10.1016/J.BIOMATERIALS.2004.10.016, vol. 26, no. 18, 1 June 2005 (2005-06-01), pages 3953-3960, XP025280550 ISSN: 0142-9612 [retrieved on 2005-06-01]
- MASTROGIACOMO M ET AL: "Bone and cartilage formation by skeletal muscle derived cells" JOURNAL OF CELLULAR PHYSIOLOGY, vol. 204, no. 2, August 2005 (2005-08), pages 594-603, XP9133039 ISSN: 0021-9541
- LEE J.Y. ET AL.: 'Clonal Isolation of Muscle-derived Cell Capable of Enhancing Muscle Regeneration and Bone Healing' JOURNAL OF CELL BIOLOGY vol. 150, no. 5, September 2000, pages 1085 - 1099, XP002161665
- SAINI A. AND STEWART C.E.: 'Adult Stem Cells: the Therapeutic Potential of Skeletal Muscle' CURR. STEM CELL RES. THER. vol. 1, no. 2, May 2006, pages 157 - 171, XP008130740

### SEQUENCE LISTING

<110> University of Pittsburgh
   Payne, Thomas
   Jankowski, Ronald
   Pruchnic, Ryan
<120> Bone Augmentation utilizing Muscle-Derived Progenitor compositions, and Treatments Thereof
<130> 28682-507001WO
<140> To be assigned
   <141> 2008-05-29
<150> 60/940576
   <151> 2007-05-29
<150> 60/972476
   <151> 2007-09-14
<160> 12
<170> PatentIn version 3.5
<210> 1
   <211> 20
   <212> DNA
   <213> Homo sapiens
<400> 1
   taacttgact tctgctacca 20
<210> 2
   <211> 20
   <212> DNA
   <213> Homo sapiens
<400> 2
   gtggtcttac tgctgtcctg 20
<210> 3
   <211> 20
   <212> DNA
   <213> Homo sapiens
<400> 3
   gaatgtcgtc ctacacggcc 20
<210> 4
   <211> 22
   <212> DNA
   <213> Homo sapiens
<400> 4
   cactacacag tcaggacact gc 22
<210> 5
   <211> 23
   <212> DNA
   <213> Homo sapiens
<400> 5
   tacttcatca aagtccctcg gtc 23
<210> 6
   <211> 24
   <212> DNA
   <213> Homo sapiens
<400> 6
   gtactctgga acagaggcta actt 24
<210> 7
   <211> 21
   <212> DNA
   <213> Homo sapiens
<400> 7
   agccctgtgc caccatgtgt c 21
<210> 8
   <211> 21
   <212> DNA
   <213> Homo sapiens
<400> 8
   ggcaggtttg tcgacctcac t 21
<210> 9
   <211> 24
   <212> DNA
   <213> Homo sapiens
<400> 9
   caaccaggag gagcgcgatc tccg 24
<210> 10
   <211> 25
   <212> DNA
   <213> Homo sapiens
<400> 10
   aggcgctgtg ggagttgcat tcact 25
<210> 11
   <211> 27
   <212> DNA
   <213> Homo sapiens
<400> 11
   gctctgatgg catgatggat tacagcg 27
<210> 12
   <211> 22
   <212> DNA
   <213> Homo sapiens
<400> 12
   atgctggaca ggcagtcgag gc 22

## Description

### FIELD OF THE INVENTION

The present invention relates to muscle-derived progenitor cells (MDCs) and compositions of MDCs and their use in the augmentation of body tissues, particularly bone. In particular, the present invention relates to muscle-derived progenitor cells that show long-term survival following introduction into bone, methods of isolating MDCs and methods of using MDC-containing compositions for the augmentation of human or animal bone. The invention also relates to novel uses of muscle-derived progenitor cells for the treatment of cosmetic or functional conditions, such as osteoporosis, Paget's Disease, osteogenesis imperfecta, bone fracture, osteomalacia, decrease in bone trabecular strength, decrease in bone cortical strength and decrease in bone density with old age. The invention also relates to the novel use of MDCs for the increase of bone mass in athletes or other organisms in need of greater than average bone mass.

### BACKGROUND OF THE INVENTION

Myoblasts, the precursors of muscle fibers, are mononucleated muscle cells that fuse to form post-mitotic multinucleated myotubes, which can provide long-term expression and delivery ofbioactive proteins (T. A. Partridge and K. E. Davies, 1995, Brit. Med. Bulletin 51:123 137; J. Dhawan et al., 1992, Science 254: 1509 12; A. D. Grinnell, 1994, Myology Ed 2, A. G. Engel and C. F. Armstrong, McGraw-Hill, Inc., 303 304; S. Jiao and J. A. Wolff, 1992, Brain Research 575:143 7; H. Vandenburgh, 1996, Human Gene Therapy 7:2195 2200).

Cultured myoblasts contain a subpopulation of cells that show some of the self renewal properties of stem cells (A. Baroffio et al., 1996, Differentiation 60:47 57). Such cells fail to fuse to form myotubes, and do not divide unless cultured separately (A. Baroffio *et al.*, *supra*). Studies of myoblast transplantation (see below) have shown that the majority of transplanted cells quickly die, while a minority survive and mediate new muscle formation (J. R. Beuchamp et al., 1999, J. Cell Biol. 144:1113 1122). This minority of cells shows distinctive behavior, including slow growth in tissue culture and rapid growth following transplantation, suggesting that these cells may represent myoblast stem cells (J. R. Beuchamp *et al.*, *supra*).

Myoblasts have been used as vehicles for gene therapy in the treatment of various muscle- and non-muscle-related disorders. For example, transplantation of genetically modified or unmodified myoblasts has been used for the treatment of Duchenne muscular dystrophy (E. Gussoni et al., 1992, Nature, 356:435 8; J. Huard et al., 1992, Muscle & Nerve, 15:550 60; G. Karpati et al., 1993, Ann. Neurol., 34:8 17; J. P. Tremblay et al., 1993, Cell Transplantation, 2:99 112; P. A. Moisset et al., 1998, Biochem. Biophys. Res. Commun. 247:94 9; P. A. Moisset et al., 1998, Gene Ther. 5:1340 46). In addition, myoblasts have been genetically engineered to produce proinsulin for the treatment of Type 1 diabetes (L. Gros et al., 1999, Hum. Gen. Ther. 10:1207 17); Factor IX for the treatment of hemophilia B (M. Roman et al., 1992, Somat. Cell. Mol. Genet. 18:247 58; S. N. Yao et al., 1994, Gen. Ther. 1:99 107; J. M. Wang et al., 1997, Blood 90:1075 82; G. Hortelano et al., 1999, Hum. Gene Ther. 10:1281 8); adenosine deaminase for the treatment of adenosine deaminase deficiency syndrome (C. M. Lynch et al., 1992, Proc. Natl. Acad. Sci. USA, 89:1138 42); erythropoietin for the treatment of chronic anemia (E. Regulier et al., 1998, Gene Ther. 5:1014 22; B. Dalle et al., 1999, Gene Ther. 6:157 61), and human growth hormone for the treatment of growth retardation (K. Anwer et al., 1998, Hum. Gen. Ther. 9:659 70).

Myoblasts have also been used to treat muscle tissue damage or disease, as disclosed in U.S. Pat. No. 5,130,141 to Law et al., U.S. Pat. No. 5,538,722 to Blau et al., and application U.S. Ser. No. 09/302,896 filed Apr. 30, 1999 by Chancellor et al. In addition, myoblast transplantation has been employed for the repair of myocardial dysfunction (C. E. Murry et al., 1996, J. Clin. Invest. 98:2512 23; B. Z. Atkins et al., 1999, Ann. Thorac. Surg. 67:124 129; B. Z. Atkins et al., 1999, J. Heart Lung Transplant. 18:1173 80).

In spite of the above, in most cases, primary myoblast-derived treatments have been associated with low survival rates of the cells following transplantation due to migration and/or phagocytosis. To circumvent this problem, U.S. Pat. No. 5,667,778 to Atala discloses the use of myoblasts suspended in a liquid polymer, such as alginate. The polymer solution acts as a matrix to prevent the myoblasts from migrating and/or undergoing phagocytosis after injection. However, the polymer solution presents the same problems as the biopolymers discussed above. Furthermore, the Atala patent is limited to uses of myoblasts in only muscle tissue, but no other tissue.

Thus, there is a need for other, different tissue augmentation materials that are long-lasting, compatible with a wide range of host tissues, and which cause minimal inflammation, scarring, and/or stiffening of the tissues surrounding the implant site. Accordingly, the muscle-derived progenitor cell (MDC)-containing compositions of the present invention are provided as improved and novel materials for augmenting bone. Further provided are methods of producing muscle-derived progenitor cell compositions that show long-term survival following transplantation, and methods of utilizing MDCs and compositions containing MDCs to treat various aesthetic and/or functional defects, including, for example osteoporosis, Paget's Disease, osteogenesis imperfecta, bone fracture, osteomalacia, decrease in bone trabecular strength, decrease in bone cortical strength and decrease in bone density with old age. Also provided are methods of using MDCs and compositions containing MDCs for the increase of bone mass in athletes or other organisms in need of greater than average bone mass.

It is notable that prior attempts to use myoblasts for non-muscle tissue augmentation were unsuccessful (U.S. Pat. No. 5,667,778 to Atala). Therefore, the findings disclosed herein are unexpected, as they show that the muscle-derived progenitor cells according to the present invention can be successfully transplanted into non-muscle tissue, including bone tissue, and exhibit long-term survival. As a result, MDCs and compositions comprising MDCs can be used as a general augmentation material for bone production. Moreover, since the muscle-derived progenitor cells and compositions of the present invention can be derived from autologous sources, they carry a reduced risk of immunological complications in the host, including the reabsorption of augmentation materials, and the inflammation and/or scarring of the tissues surrounding the implant site.

Although mesenchymal stem cells can be found in various connective tissues of the body including muscle, bone, cartilage, *etc.* (H. E. Young et al., 1993, In vitro Cell Dev. Biol. 29A:723 736; H. E. Young, et al., 1995, Dev. Dynam. 202:137 144), the term mesenchymal has been used historically to refer to a class of stem cells purified from bone marrow, and not from muscle. Thus, mesenchymal stem cells are distinguished from the muscle-derived progenitor cells of the present invention. Moreover, mesenchymal cells do not express the CD34 cell marker (M. F. Pittenger et al., 1999, Science 284:143 147), which is expressed by the muscle-derived progenitor cells described herein.

The description herein of disadvantages and problems associated with known compositions, and methods is in no way intended to limit the scope of the embodiments described in this document to their exclusion. Indeed, certain embodiments may include one or more known compositions, compounds, or methods without suffering from the so-noted disadvantages or problems.

### SUMMARY OF THE INVENTION

It is an object of the present invention to provide novel muscle-derived progenitor cells (MDCs) and MDC compositions exhibiting long-term survival following transplantation. The MDCs of this invention and compositions containing the MDCs comprise early progenitor muscle cells, i.e., muscle-derived stem cells that express progenitor cell markers, such as desmin, M-cadherin, MyoD, myogenin, CD34, and Bcl-2. In addition, these early progenitor muscle cells express the Flk-1, Sca-1, MNF, and c-met cell markers, but do not express the CD45 or c-Kit cell markers.

It is another object of the present invention to provide methods for isolating and enriching muscle-derived progenitor cells from a starting muscle cell population. These methods result in the enrichment of MDCs that have long-term survivability after transplantation or introduction into a site of soft tissue. The MDC population according to the present invention is particularly enriched with cells that express progenitor cell markers, such as desmin, M-cadherin, MyoD, myogenin, CD34, and Bcl-2. This MDC population also expresses the Flk-1, Sca-1, MNF, and c-met cell markers, but does not express the CD45 or c-Kit cell markers.

It is yet another object of the present invention to provide methods of using MDCs and compositions comprising MDCs for the augmentation of non-muscle tissue, including bone, without the need for polymer carriers or special culture media for transplantation. Such methods include the administration of MDC compositions by introduction into bone, for example by direct injection into or on the surface of the tissue, or by systemic distribution of the compositions.

It is yet another object of the present invention to provide methods of augmenting bone, following injury, wounding, surgeries, traumas, non-traumas, or other procedures that result in fissures, openings, depressions, wounds, and the like.

It is a further object of the present invention to provide MDCs and compositions comprising MDCs that are modified through the use of chemicals, growth media, and/or genetic manipulation. Such MDCs and compositions thereof comprise chemically or genetically modified cells useful for the production and delivery of biological compounds, and the treatment of various diseases, conditions, injuries, or illnesses.

It is a further object of the present invention to provide MDCs and compositions comprising MDCs that are modified through the use of chemicals, growth media, and/or genetic manipulation. Such MDCs and compositions thereof comprise chemically or genetically modified cells useful for the production and delivery of biological compounds, and the treatment of various diseases, conditions, injuries, or illnesses.

It is yet another embodiment of the invention to provide pharmaceutical compositions comprising MDCs and compositions comprising MDCs. These pharmaceutical compositions comprise isolated MDCs. These MDCs may be subsequently expanded by cell culture after isolation. On one aspect of this embodiment, these MDCs are frozen prior to delivery to a subject in need of the pharmaceutical composition.

The invention also provides compositions and methods involving the isolation of MDCs using a single plating technique. MDCs are isolated from a biopsy of skeletal muscle. In one embodiment, the skeletal muscle from the biopsy may be stored for 1-6 days. In one aspect of this embodiment, the skeletal muscle from the biopsy is stored at 4 °C. The cells are minced, and digested using a collagenase, dispase, another enzyme or a combination of enzymes. After washing the enzyme from the cells, the cells are cultured in a flask in culture medium for between about 30 and about 120 minutes. During this period of time, the "rapidly adhering cells" stick to the walls of the flask or container, while the "slowly adhering cells" or MDCs remain in suspension. The "slowly adhering cells" are transferred to a second flask or container and cultured therein for a period of 1-3 days. During this second period of time the "slowly adhering cells" or MDCs stick to the walls of the second flask or container.

In another embodiment of the invention, these MDCs are expanded to any number of cells. In a preferred aspect of this embodiment, the cells are expanded in new culture media for between about 10 and 20 days. More preferably, the cells are expanded for 17 days.

The MDCs, whether expanded or not expanded, may be preserved in order to be transported or stored for a period of time before use. In one embodiment, the MDCs are frozen. Preferably, the MDCs are frozen at between about -20 and -90 °C. More preferably, the MDCs are frozen at about -80 °C. These frozen MDCs are used as a pharmaceutical composition.

MDCs, whether frozen or preserved as a pharmaceutical composition, or used fresh, may be used to treat a number of bone degenerative diseases, defects and pathologies. These conditions include osteoporosis, Paget's Disease, osteogenesis imperfecta, bone fracture, osteomalacia, decrease in bone trabecular strength, decrease in bone cortical strength and decrease in bone density with old age. MDCs, whether frozen or preserved as a pharmaceutical composition, or used fresh, may also be used for the increase of bone mass in athletes or other organisms in need of greater than average bone mass.

Further, the invention provides a method of treating a bone disease, defect or pathology or augmenting bone mass or density in a mammalian subject in need thereof. The method comprises isolating skeletal muscle cells from a mammal; cooling the cells to a temperature lower than 10 °C and storing the cells for 1-7 days; suspending the mammalian skeletal muscle cells in a first cell culture container between 30 and 120 minutes; decanting the media from the first cell culture container to a second cell culture container; allowing the remaining cells in the media to attach to the walls of the second cell culture container; isolating the cells from the walls of the second cell culture container, wherein the isolated cells are muscle derived progenitor cells (MDCs); culturing the cells to expand their number; freezing the MDCs to a temperature below -30 °C; and thawing the MDCs and administering the MDCs to a bone suffering from the bone defect, disease or pathology of the mammalian subject; thereby, treating bone defect, disease or pathology in the mammalian subject in need thereof.

The invention also provides a method of improving at least one symptom associated with bone disease, defect or pathology in a mammalian subject in need thereof. The method comprises: isolating skeletal muscle cells from a mammal; suspending the mammalian skeletal muscle cells in a first cell culture container for between 30 and 120 minutes; decanting the media from the first cell culture container to a second cell culture container; allowing the remaining cells in the media to attach to the walls of the second cell culture container; isolating the cells from the walls of the second cell culture container, wherein the isolated cells are MDCs; and administering the MDCs to a bone suffering from the bone defect, disease or pathology of the mammalian subject; thereby, improving at least one symptom associated with bone disease, defect or pathology in a mammalian subject in need thereof.

The invention also provides a method of treating a bone disease, defect or pathology or improving at least one symptom associated with bone disease, defect or pathology in a mammalian subject in need thereof. The method comprises: plating a suspension of skeletal muscle cells from human skeletal muscle tissue in a first container to which fibroblast cells of the skeletal muscle cell suspension adhere; re-plating non-adherent cells from step (a) in a second container, wherein the step of re-plating is after 15-20% of cells have adhered to the first container; (c) repeating step (b) at least once; (d) isolating the skeletal muscle-derived MDCs and administering the MDCs to a bone suffering from the bone defect, disease or pathology of the mammalian subject; thereby, treating urinary tract disease in a mammalian subject in need thereof.

The invention also provides a method of treating a bone defect, disease or pathology in a mammalian subject in need thereof. The method comprises: administering a cell population containing muscle-derived cells (MDCs) to a bone suffering from the bone defect, disease or pathology of the mammalian subject. The cell population containing MDCs is obtained by a process comprising: suspending cells isolated from mammalian skeletal muscle in a first cell culture container for a duration sufficient to adhere a first cell population to the container and to leave a second cell population unadhered and in a culture medium in the container; transferring the culture medium and second cell population from the first cell culture container to a second cell culture container; allowing cells from the second cell population to attach to the second cell culture container; and isolating the cells attached to the second cell culture container to obtain said cell population containing MDCs.

Additional objects and advantages afforded by the present invention will be apparent from the detailed description and exemplification hereinbelow.

### BRIEF DESCRIPTION OF THE DRAWINGS

The patent or patent application file contains at least one photographic reproductions executed in color. Copies of this patent or patent application with color photographic reproduction(s) will be provided by the U.S. Patent and Trademark Office upon request and payment of the necessary fee.

The appended drawings of the figures are presented to further describe the invention and to assist in its understanding through clarification of its various aspects.

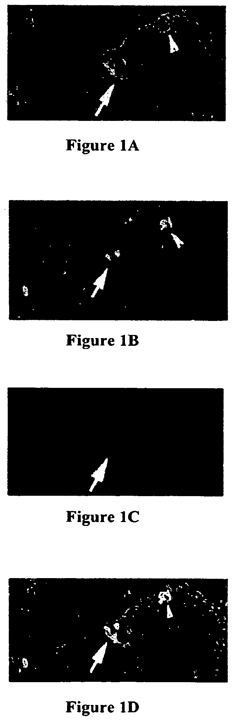

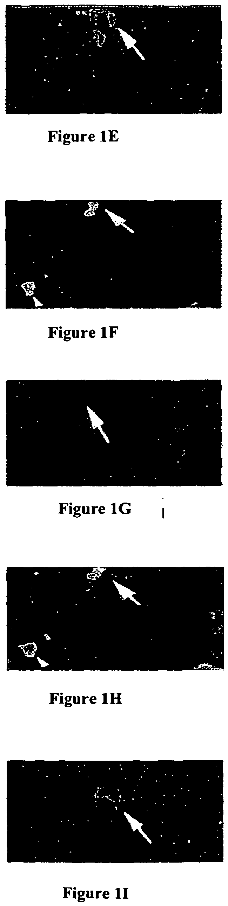

Figures 1A-1I illustrate the intracellular co-localization of CD34 or Bcl-2 staining with desmin staining in mouse muscle cells and vascular endothelial cells. Figure 1A shows normal mouse muscle cells (see arrow) and vascular endothelial cells (see arrowhead) stained with anti-CD34 antibodies and visualized by fluorescence microscopy. Figure 1B shows the same cells co-stained with desmin and collagen type IV antibodies. Figure 1C shows the same cells co-stained with Hoechst to show the nuclei. Figure 1D shows a composite of the cells co-stained for CD34, desmin, collagen type IV, and Hoechst. Figure 1E shows normal mouse muscle cells (see arrow) stained with anti-Bcl-2 antibodies and visualized by fluorescence microscopy. Figure 1F shows the same cells co-stained with desmin and collagen type IV antibodies. Figure 1G shows the same cells co-stained with Hoechst to show the nuclei. Figure 1H shows a composite of the cells co-stained for CD34, desmin, collagen type IV, and Hoechst. Figure 1I shows satellite cells stained with anti-M-cadherin antibodies (see arrow). Cells were viewed at 40x magnification. Figure 1A-1D demonstrate the co-localization of CD34 and desmin, while Figure 1E-1H demonstrate the co-localization of Bcl-2 and desmin.

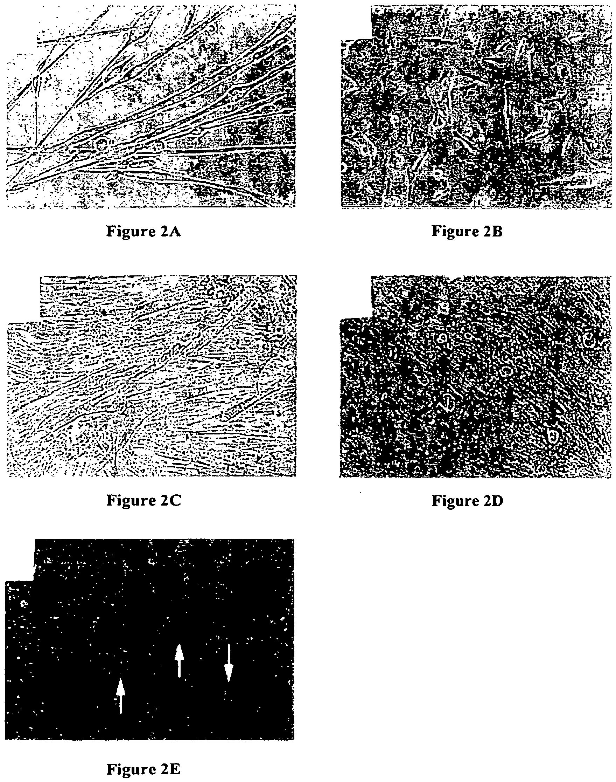

Figures 2A-2E illustrate the morphologic changes and expression of osteocalcin resulting from the exposure of mc13 cells to rhBMP-2. Mc13 cells were incubated in growth media with or without rhBMP-2 for 6 days. Figure 2A shows cells grown to >50% cell confluency in the absence of rhBMP-2. Figure 2B shows cells grown to >50% cell confluency in the presence of 200 ng/ml rhBMP-2. Figure 2C shows cells grown to >90% cell confluency in the absence of rhBMP-2. Figure 2D shows cells grown to >90% confluency in the presence of 200 ng/ml rhBMP-2. Figure 2E shows cells stained for osteocalcin expression (osteoblast cell marker; see arrows). Cells were viewed at 10x magnification. Figure 2A-2E demonstrate that mc13 cells are capable of differentiating into osteoblasts upon exposure to rhBMP-2.

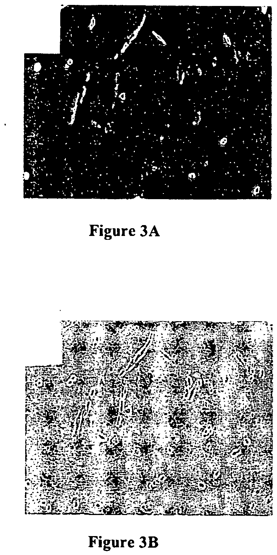

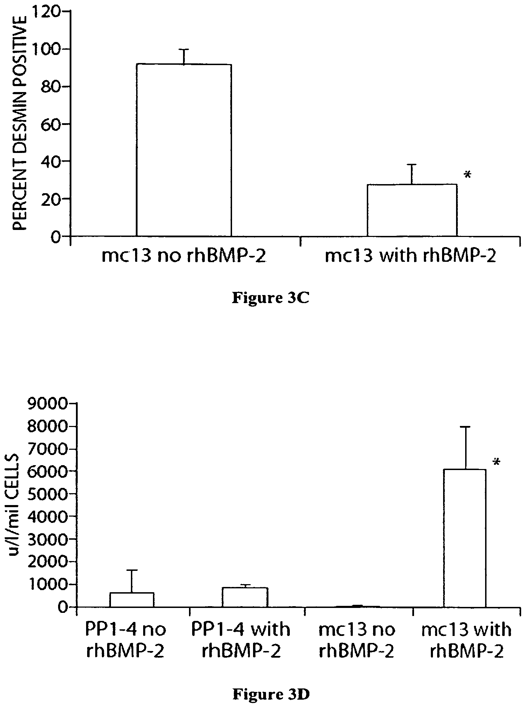

Figures 3A-3D illustrate the effects on the percentage of mc13 cells expressing desmin and alkaline phosphatase in response to rhBMP-2 treatment. Figure 3A shows desmin staining of newly isolated mc13 clones. Figure 3B shows a phase contrast view of the same cells. Figure 3C shows the levels of desmin staining in mc13 cells following 6 days of incubation in growth media with or without 200 ng/ml rhBMP-2. Figure 3D shows the levels of alkaline phosphate staining in PP14 cells and mc13 cells following 6 days of incubation in growth media with or without 200 ng/ml rhBMP-2. * indicates a statistically significant result (student's t-test). Figure 3C demonstrates that a decreasing number of mc13 cells express desmin in the presence of rhBMP-2, while Figure 3D demonstrates that an increasing number of mc13 cells express alkaline phosphatase in the presence of rhBMP-2, suggesting decreasing myogenic characteristics and increasing osteogenic characteristics of the cells in the presence of rhBMP-2.

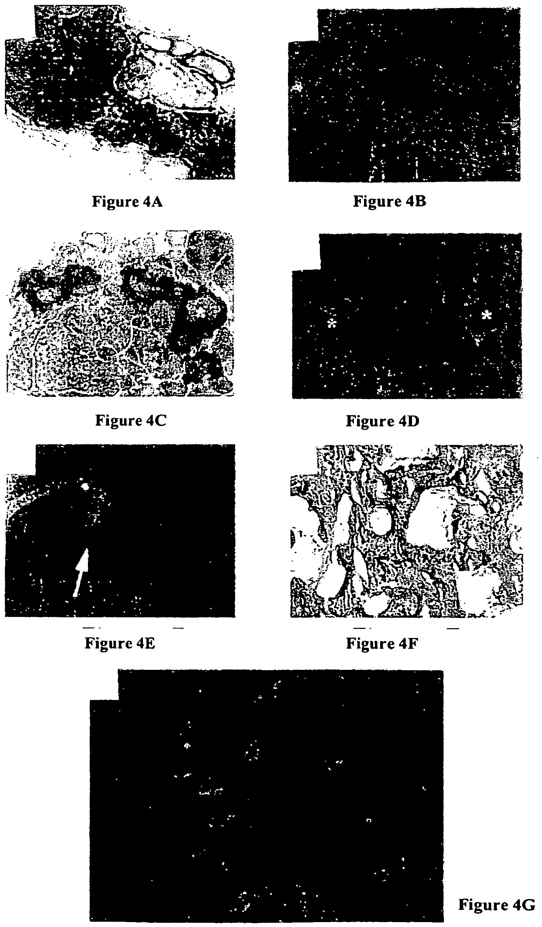

Figures 4A-4G illustrate the *in vivo* differentiation of mc13 cells into myogenic and osteogenic lineages. Mc13 cells were stably transfected with a construct containing LacZ and the dystrophin gene, and introduced by intramuscular or intravenous injection into hind limbs of mdx mice. After 15 days, the animals were sacrificed and the hind limb musculature was isolated for histology. Figure 4A shows mc13 cells at the intramuscular injection site stained for LacZ. Figure 4B shows the same cells co-stained for dystrophin. Figure 4C shows mc 13 cells in the region of the intravenous injection stained for LacZ. Figure 4D shows the same cells co-stained for dystrophin. In a separate experiment, mc 13 cells were transduced with adBMP-2, and 0.5 1.0x10⁶ cells were injected into hind limbs of SCID mice. After 14 days, the animals were sacrificed, and the hind limb muscle tissues were analyzed. Figure 4E shows radiographic analysis of the hind limb to determine bone formation. Figure 4F shows the cells derived from the hind limb stained for LacZ. Figure 4G shows cells stained for dystrophin. Figures 4A-4D demonstrate that mc13 cells can rescue dystrophin expression via intramuscular or intravenous delivery. Figures 4A-4G demonstrate that mc13 cells are involved in ectopic bone formation. Cells were viewed at the following magnifications: 40x (Figures 4A-4D); 10x (Figures 4A-4G).

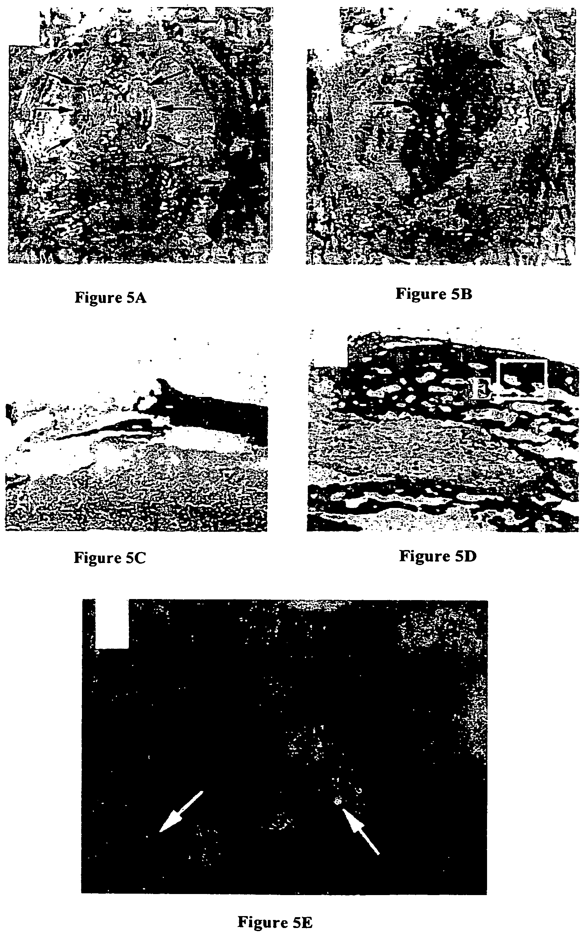

Figures 5A-5E illustrate the enhancement of bone healing by rhBMP-2 producing primary muscle cells. A 5 mm skull defect was created in female SCID mice using a dental burr, and the defect was filled with a collagen sponge seeded with mc13 cells with or without adBMP-2. The animals were sacrificed at 14 days, inspected, and analyzed microscopically for indications of bone healing. Figure 5A shows a skull treated with mc13 cells without adBMP-2. Figure 5B shows a skull treated with mc13 cells transduced with adBMP-2. Figure 5C shows a histological sample of the skull treated with mc13 cells without adBMP-2 analyzed by von Kossa staining. Figure 5D shows a histological sample of the skull treated with mc13 cells transduced with adBMP-2 analyzed by von Kossa staining. Figure 5E shows a histological sample of the skull treated with the mc13 cells transduced with adBMP-2 analyzed by hybridization with a Y-chromosome specific probe to identify the injected cells (green fluorescence shown by arrows), and stained with ethidium bromide to identify the nuclei (indicated by red fluorescence). Figures 5A-5E demonstrate that mc13 cells expressing rhBMP-2 can contribute to the healing of bone defects.

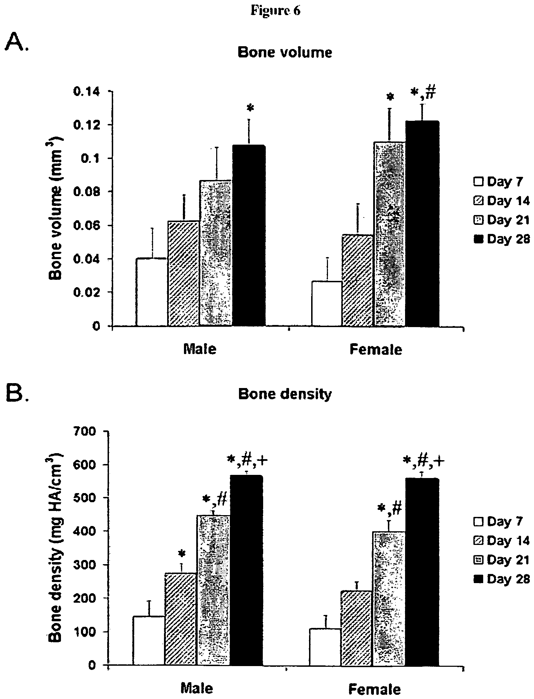

Figures 6A and 6B are bar graphs showing bone volume (Figure 6A) and bone density (Figure 6B) increasing over time in osteogenic pellets comprising human male and female MDCs in OSM. *P<0.05 vs. Day 7, #P<0.05 vs. Day 14, and +P<0.05 vs. Day 21.

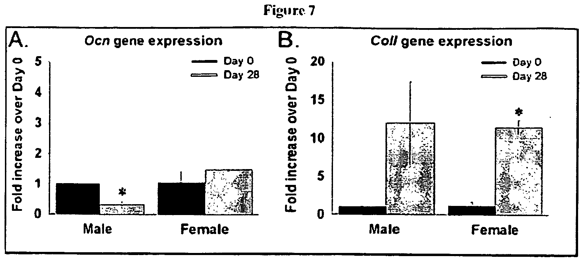

Figures 7A and 7B are bar graphs showing Osteocalcin (Ocn) (Figure 7A) and Collagen type I (ColI) (Figure 7B) gene expression of hMDCs cultured as pellets in OSM. *P<0.05 vs. Day 0.

### DETAILED DESCRIPTION OF THE INVENTION

The invention provides human MDCs and methods of using such cells to generate bone tissue to repair damaged bone or to increase bone volume and/or density to above wild type levels. The invention further provides methods of treating bone disorders including incontinence osteoporosis, Paget's Disease, osteogenesis imperfecta, bone fracture, osteomalacia, decrease in bone trabecular strength, decrease in bone cortical strength and decrease in bone density with old age. The isolation of human muscle-derived cells (MDCs) from adult tissue are capable of achieving increased bone density and bone volume within human subjects administered these cells.

### Muscle-Derived Cells and Compositions

The present invention provides MDCs comprised of early progenitor cells (also termed muscle-derived progenitor cells or muscle-derived stem cells herein) that show long-term survival rates following transplantation into body tissues, preferably bone. To obtain the MDCs of this invention, a muscle explant, preferably skeletal muscle, is obtained from an animal donor, preferably from a mammal, including humans. This explant serves as a structural and functional syncytium including "rests" of muscle precursor cells (T. A. Partridge et al., 1978, Nature 73:306 8; B. H. Lipton et al., 1979, Science 205:12924).

Cells isolated from primary muscle tissue contain mixture of fibroblasts, myoblasts, adipocytes, hematopoietic, and muscle-derived progenitor cells. The progenitor cells of a muscle-derived population can be enriched using differential adherence characteristics of primary muscle cells on collagen coated tissue flasks, such as described in U.S. Pat. No. 6,866,842 of Chancellor et al. Cells that are slow to adhere tend to be morphologically round, express high levels of desmin, and have the ability to fuse and differentiate into multinucleated myotubes U.S. Pat. No. 6,866,842 of Chancellor et al.). A subpopulation of these cells was shown to respond to recombinant human bone morphogenic protein 2 (rhBMP-2) *in vitro* by expressing increased levels of alkaline phosphatase, parathyroid hormone dependent 3',5'-cAMP, and osteogenic lineage and myogenic lineages (U.S. Pat. No. 6,866,842 of Chancellor et al.; T. Katagiri et al., 1994, J. Cell Biol., 127:1755 1766).

In one embodiment of the invention, a preplating procedure may be used to differentiate rapidly adhering cells from slowly adhering cells (MDCs). In accordance with the present invention, populations of rapidly adhering MDC (PP1-4) and slowly adhering, round MDC (PP6) were isolated and enriched from skeletal muscle explants and tested for the expression of various markers using immunohistochemistry to determine the presence of pluripotent cells among the slowly adhering cells (Example 1; patent application U.S. Ser. No. 09/302,896 of Chancellor et al.). As shown in Table 2, Example 3 herein, the PP6 cells expressed myogenic markers, including desmin, MyoD, and Myogenin. The PP6 cells also expressed c-met and MNF, two genes which are expressed at an early stage of myogenesis (J. B. Miller et al., 1999, Curr. Top. Dev. Biol. 43:191 219; see Table 3). The PP6 showed a lower percentage of cells expressing M-cadherin, a satellite cell-specific marker (A. Irintchev et al., 1994, Development Dynamics 199:326 337), but a higher percentage of cells expressing Bcl-2, a marker limited to cells in the early stages of myogenesis (J. A. Dominov et al., 1998, J. Cell Biol. 142:537 544). The PP6 cells also expressed CD34, a marker identified with human hematopoietic progenitor cells, as well as stromal cell precursors in bone marrow (R. G. Andrews et al., 1986, Blood 67:842 845; C. I. Civin et al., 1984, J. Immunol. 133:157 165; L. Fina et al, 1990, Blood 75:2417 2426; P. J. Simmons et al., 1991, Blood 78:2848 2853; see Table 3). The PP6 cells also expressed Flk-1, a mouse homologue of human KDR gene which was recently identified as a marker of hematopoietic cells with stem cell-like characteristics (B. L. Ziegler et al., 1999, Science 285:1553 1558; see Table 3). Similarly, the PP6 cells expressed Sca-1, a marker present in hematopoietic cells with stem cell-like characteristics (M. van de Rijn et al., 1989, Proc. Natl. Acad. Sci. USA 86:4634 8; M. Osawa et al., 1996, J. Immunol. 156:3207 14; see Table 3). However, the PP6 cells did not express the CD45 or c-Kit hematopoietic stem cell markers (reviewed in L K. Ashman, 1999, Int. J. Biochem. Cell. Biol. 31:1037 51; G. A. Koretzky, 1993, FASEB J. 7:420 426; see Table 3).

In one embodiment of the present invention is the PP6 population of muscle-derived progenitor cells having the characteristics described herein. These muscle-derived progenitor cells express the desmin, CD34, and Bcl-2 cell markers. In accordance with the present invention, the PP6 cells are isolated by the techniques described herein (Example 1) to obtain a population of muscle-derived progenitor cells that have long-term survivability following transplantation. The PP6 muscle-derived progenitor cell population comprises a significant percentage of cells that express progenitor cell markers such as desmin, CD34, and Bcl-2. In addition, PP6 cells express the Flk-I and Sca-1 cell markers, but do not express the CD45 or c-Kit markers. Preferably, greater than 95% of the PP6 cells express the desmin, Sca-1, and Flk-1 markers, but do not express the CD45 or c-Kit markers. It is preferred that the PP6 cells are utilized within about 1 day or about 24 hours after the last plating.

In a preferred embodiment, the rapidly adhering cells and slowly adhering cells (MDCs) are separated from each other using a single plating technique. One such technique is described in Example 2. First, cells are provided from a skeletal muscle biopsy. The biopsy need only contain about 100 mg of cells. Biopsies ranging in size from about 50 mg to about 500 mg are used according to both the pre-plating and single plating methods of the invention. Further biopsies of 50, 100, 110, 120, 130, 140, 150, 200, 250, 300, 400 and 500 mg are used according to both the pre-plating and single plating methods of the invention.

In a preferred embodiment of the invention, the tissue from the biopsy is then stored for 1 to 7 days. This storage is at a temperature from about room temperature to about 4 °C. This waiting period causes the biopsied skeletal muscle tissue to undergo stress. While this stress is not necessary for the isolation of MDCs using this single plate technique, it seems that using the wait period results in a greater yield of MDCs.

According to preferred embodiments, tissue from the biopsies is minced and centrifuged. The pellet is resuspended and digested using a digestion enzyme. Enzymes that may be used include collagenase, dispase or combinations of these enzymes. After digestion, the enzyme is washed off of the cells. The cells are transferred to a flask in culture media for the isolation of the rapidly adhering cells. Many culture media may be used. Particularly preferred culture media include those that are designed for culture of endothelial cells including Cambrex Endothelial Growth Medium. This medium may be supplemented with other components including fetal bovine serum, IGF-1, bFGF, VEGF, EGF, hydrocortisone, heparin, and/or ascorbic acid. Other media that may be used in the single plating technique include InCell M310F medium. This medium may be supplemented as described above, or used unsupplemented.

The step for isolation of the rapidly adhering cells may require culture in flask for a period of time from about 30 to about 120 minutes. The rapidly adhering cells adhere to the flask in 30, 40, 50, 60, 70, 80, 90, 100, 110 or 120 minutes. After they adhere, the slowly adhering cells are separated from the rapidly adhering cells from removing the culture media from the flask to which the rapidly adhering cells are attached to.

The culture medium removed from this flask is then transferred to a second flask. The cells may be centrifuged and resuspended in culture medium before being transferred to the second flask. The cells are cultured in this second flask for between 1 and 3 days. Preferably, the cells are cultured for two days. During this period of time, the slowly adhering cells (MDCs) adhere to the flask. After the MDCs have adhered, the culture media is removed and new culture media is added so that the MDCs can be expanded in number. The MDCs may be expanded in number by culturing them for from about 10 to about 20 days. The MDCs may be expanded in number by culturing them for 10, 11, 12, 13, 14, 15, 16, 17, 18, 19 or 20 days. Preferably, the MDCs are subject to expansion culture for 17 days.

As an alternative to the pre-plating and single plating methods, the MDCs of the present invention can be isolated by fluorescence-activated cell sorting (FACS) analysis using labeled antibodies against one or more of the cell surface markers expressed by the MDCs (C. Webster et al., 1988, Exp. Cell. Res. 174:252 65; J. R. Blanton et al., 1999, Muscle Nerve 22:43 50). For example, FACS analysis can be performed using labeled antibodies that specifically bind to CD34, Flk-1, Sca-1, and/or the other cell-surface markers described herein to select a population of PP6-like cells that exhibit long-term survivability when introduced into the host tissue. Also encompassed by the present invention is the use of one or more fluorescence-detection labels, for example, fluorescein or rhodamine, for antibody detection of different cell marker proteins.

Using any of the MDCs isolation methods described above, MDCs that are to be transported, or are not going to be used for a period of time may be preserved using methods known in the art. More specifically, the isolated MDCs may be frozen to a temperature ranging from about -25 to about -90 °C. Preferably, the MDCs are frozen at about -80 °C, on dry ice for delayed use or transport. The freezing may be done with any cryopreservation medium known in the art.

### Muscle-Derived Cell-Based Treatments

In one embodiment of the present invention, the MDCs are isolated from a skeletal muscle source and introduced or transplanted into a muscle or non-muscle soft tissue site of interest, or into bone structures. Advantageously, the MDCs of the present invention are isolated and enriched to contain a large number of progenitor cells showing long-term survival following transplantation. In addition, the muscle-derived progenitor cells of this invention express a number of characteristic cell markers, such desmin, CD34, and Bcl-2. Furthermore, the muscle-derived progenitor cells of this invention express the Sca-1, and Flk-1 cell markers, but do not express the CD45 or c-Kit cell markers (see Example 1).

MDCs and compositions comprising MDCs of the present invention can be used to repair, treat, or ameliorate various aesthetic or functional conditions (e.g. defects) through the augmentation of bone. In particular, such compositions can be used for the treatment of bone disorders. Multiple and successive administrations of MDC are also embraced by the present invention.

For MDC-based treatments, a skeletal muscle explant is preferably obtained from an autologous or heterologous human or animal source. An autologous animal or human source is more preferred. MDC compositions are then prepared and isolated as described herein. To introduce or transplant the MDCs and/or compositions comprising the MDCs according to the present invention into a human or animal recipient, a suspension of mononucleated muscle cells is prepared. Such suspensions contain concentrations of the muscle-derived progenitor cells of the invention in a physiologically-acceptable carrier, excipient, or diluent. For example, suspensions of MDC for administering to a subject can comprise 10⁸ to 10⁹ cells/ml in a sterile solution of complete medium modified to contain the subject's serum, as an alternative to fetal bovine serum. Alternatively, MDC suspensions can be in serum-free, sterile solutions, such as cryopreservation solutions (Celox Laboratories, St. Paul, Minn.). The MDC suspensions can then be introduced *e.g.*, via injection, into one or more sites of the donor tissue.

The described cells can be administered as a pharmaceutically or physiologically acceptable preparation or composition containing a physiologically acceptable carrier, excipient, or diluent, and administered to the tissues of the recipient organism of interest, including humans and non-human animals. The MDC-containing composition can be prepared by resuspending the cells in a suitable liquid or solution such as sterile physiological saline or other physiologically acceptable injectable aqueous liquids. The amounts of the components to be used in such compositions can be routinely determined by those having skill in the art.

The MDCs or compositions thereof can be administered by placement of the MDC suspensions onto absorbent or adherent material, *i.e.*, a collagen sponge matrix, and insertion of the MDC-containing material into or onto the site of interest. Alternatively, the MDCs can be administered by parenteral routes of injection, including subcutaneous, intravenous, intramuscular, and intrasternal. Other modes of administration include, but are not limited to, intranasal, intrathecal, intracutaneous, percutaneous, enteral, and sublingual. In one embodiment of the present invention, administration of the MDCs can be mediated by endoscopic surgery.

For injectable administration, the composition is in sterile solution or suspension or can be resuspended in pharmaceutically- and physiologically-acceptable aqueous or oleaginous vehicles, which may contain preservatives, stabilizers, and material for rendering the solution or suspension isotonic with body fluids (*i.e.* blood) of the recipient. Non-limiting examples of excipients suitable for use include water, phosphate buffered saline, pH 7.4, 0.15 M aqueous sodium chloride solution, dextrose, glycerol, dilute ethanol, and the like, and mixtures thereof. Illustrative stabilizers are polyethylene glycol, proteins, saccharides, amino acids, inorganic acids, and organic acids, which may be used either on their own or as admixtures. The amounts or quantities, as well as the routes of administration used, are determined on an individual basis, and correspond to the amounts used in similar types of applications or indications known to those of skill in the art.

To optimize transplant success, the closest possible immunological match between donor and recipient is desired. If an autologous source is not available, donor and recipient Class I and Class II histocompatibility antigens can be analyzed to determine the closest match available. This minimizes or eliminates immune rejection and reduces the need for immunosuppressive or immunomodulatory therapy. If required, immunosuppressive or immunomodulatory therapy can be started before, during, and/or after the transplant procedure. For example, cyclosporin A or other immunosuppressive drugs can be administered to the transplant recipient. Immunological tolerance may also be induced prior to transplantation by alternative methods known in the art (D. J. Watt et al., 1984, Clin. Exp. Immunol. 55:419; D. Faustman et al., 1991, Science 252:1701).

Consistent with the present invention, the MDCs can be administered to body tissues, including bone. The number of cells in an MDC suspension and the mode of administration may vary depending on the site and condition being treated. From about 1.0x10⁵ to about 1x10⁸ MDCs may be administered according to the invention. As a non-limiting example, in accordance with the present invention, about 0.5-1.0x10⁶ MDCs are administered via a collagen sponge matrix for the treatment of an approximately 5 mm region of skull defect (see Example 3). Further MDCs may be administered via a pellet based culture system with between about 100,000 and 500,000 MDCs per pellet. In a preferred embodiment, each pellet contains about 250,000 MDCs. Any number of pellets may be administered to a patient. Preferably between 20 two and 10 pellets are administered. Consistent with the Examples disclosed herein, a skilled practitioner can modulate the amounts and methods of MDC-based treatments according to requirements, limitations, and/or optimizations determined for each case.

For bone augmentation or treatment of bone disorders, the MDCs are prepared as described above and are administered, e.g. via injection, onto, into or around bone tissue to provide additional bone density and/or volume. As is appreciated by the skilled practitioner, the number of MDC introduced is modulated to provide varying amounts of bone density and/or bone volume, as needed or required. For example, about 0.5-1.5x10⁶ MDCs are injected for the augmentation of bone (see Example 3). Thus, the present invention also embraces the use of MDC of the invention in treating bone disorders or enhancing bone density and/or bone volume. Bone disorders osteoporosis, Paget's Disease, osteogenesis imperfecta, bone fracture, osteomalacia, decrease in bone trabecular strength, decrease in bone cortical strength and decrease in bone density with old age. The invention also relates to the novel use of MDCs for the increase of bone mass in athletes or other organisms in need of greater than average bone mass.

### Genetically Engineered Muscle-Derived Cells

In another aspect of the present invention, the MDCs of this invention may be genetically engineered to contain a nucleic acid sequence(s) encoding one or more active biomolecules, and to express these biomolecules, including proteins, polypeptides, peptides, hormones, metabolites, drugs, enzymes, and the like. Such MDCs may be histocompatible (autologous) or nonhistocompatible (allogeneic) to the recipient, including humans. These cells can serve as long-term local delivery systems for a variety of treatments, for example, for the treatment of bone diseases and pathologies, including, but not limited to osteoporosis, Paget's Disease, osteogenesis imperfecta, bone fracture, osteomalacia, decrease in bone trabecular strength, decrease in bone cortical strength and decrease in bone density with old age.

Preferred in the present invention are autologous muscle-derived progenitor cells, which will not be recognized as foreign to the recipient. In this regard, the MDC used for cell-mediated gene transfer or delivery will desirably be matched vis-a-vis the major histocompatibility locus (MHC or HLA in humans). Such MHC or HLA matched cells may be autologous. Alternatively, the cells may be from a person having the same or a similar MHC or HLA antigen profile. The patient may also be tolerized to the allogeneic MHC antigens. The present invention also encompasses the use of cells lacking MHC Class I and/or II antigens, such as described in U.S. Pat. No. 5,538,722, incorporated herein by reference.

The MDCs may be genetically engineered by a variety of molecular techniques and methods known to those having skill in the art, for example, transfection, infection, or transduction. Transduction as used herein commonly refers to cells that have been genetically engineered to contain a foreign or heterologous gene via the introduction of a viral or non-viral vector into the cells. Transfection more commonly refers to cells that have been genetically engineered to contain a foreign gene harbored in a plasmid, or non-viral vector. MDCs can be transfected or transduced by different vectors and thus can serve as gene delivery vehicles to transfer the expressed products into muscle.

Although viral vectors are preferred, those having skill in the art will appreciate that the genetic engineering of cells to contain nucleic acid sequences encoding desired proteins or polypeptides, cytokines, and the like, may be carried out by methods known in the art, for example, as described in U.S. Pat. No. 5,538,722, including fusion, transfection, lipofection mediated by the use of liposomes, electroporation, precipitation with DEAE-Dextran or calcium phosphate, particle bombardment (biolistics) with nucleic acid-coated particles (e.g., gold particles), microinjection, and the like.

Vectors for introducing heterologous (*i.e.*, foreign) nucleic acid (DNA or RNA) into muscle cells for the expression of bioactive products are well known in the art. Such vectors possess a promoter sequence, preferably, a promoter that is cell-specific and placed upstream of the sequence to be expressed. The vectors may also contain, optionally, one or more expressible marker genes for expression as an indication of successful transfection and expression of the nucleic acid sequences contained in the vector.

Illustrative examples of vehicles or vector constructs for transfection or infection of the muscle-derived cells of the present invention include replication-defective viral vectors, DNA virus or RNA virus (retrovirus) vectors, such as adenovirus, herpes simplex virus and adeno-associated viral vectors. Adeno-associated virus vectors are single stranded and allow the efficient delivery of multiple copies of nucleic acid to the cell's nucleus. Preferred are adenovirus vectors. The vectors will normally be substantially free of any prokaryotic DNA and may comprise a number of different functional nucleic acid sequences. Examples of such functional sequences include polynucleotide, *e.g.*, DNA or RNA, sequences comprising transcriptional and translational initiation and termination regulatory sequences, including promoters (*e.g.*, strong promoters, inducible promoters, and the like) and enhancers which are active in muscle cells.

Also included as part of the functional sequences is an open reading frame (polynucleotide sequence) encoding a protein of interest; flanking sequences may also be included for site-directed integration. In some situations, the 5'-flanking sequence will allow homologous recombination, thus changing the nature of the transcriptional initiation region, so as to provide for inducible or noninducible transcription to increase or decrease the level of transcription, as an example.

In general, the nucleic acid sequence desired to be expressed by the muscle-derived progenitor cell is that of a structural gene, or a functional fragment, segment or portion of the gene, that is heterologous to the muscle-derived progenitor cell and encodes a desired protein or polypeptide product, for example. The encoded and expressed product may be intracellular, *i.e.*, retained in the cytoplasm, nucleus, or an organelle of a cell, or may be secreted by the cell. For secretion, the natural signal sequence present in the structural gene may be retained, or a signal sequence that is not naturally present in the structural gene may be used. When the polypeptide or peptide is a fragment of a protein that is larger, a signal sequence may be provided so that, upon secretion and processing at the processing site, the desired protein will have the natural sequence. Examples of genes of interest for use in accordance with the present invention include genes encoding cell growth factors, cell differentiation factors, cell signaling factors and programmed cell death factors. Specific examples include, but are not limited to, genes encoding BMP-2 (rhBMP-2), IL-1Ra, Factor IX, and connexin 43.

As mentioned above, a marker may be present for selection of cells containing the vector construct. The marker may be an inducible or non-inducible gene and will generally allow for positive selection under induction, or without induction, respectively. Examples of commonly-used marker genes include neomycin, dihydrofolate reductase, glutamine synthetase, and the like.

The vector employed will generally also include an origin of replication and other genes that are necessary for replication in the host cells, as routinely employed by those having skill in the art. As an example, the replication system comprising the origin of replication and any proteins associated with replication encoded by a particular virus may be included as part of the construct. The replication system must be selected so that the genes encoding products necessary for replication do not ultimately transform the muscle-derived cells. Such replication systems are represented by replication-defective adenovirus constructed as described, for example, by G. Acsadi et al., 1994, Hum. Mol. Genet 3:579 584, and by Epstein-Barr virus. Examples of replication defective vectors, particularly, retroviral vectors that are replication defective, are BAG, described by Price et al., 1987, Proc. Natl. Acad. Sci. USA, 84:156; and Sanes et al., 1986, EMBO J., 5:3133. It will be understood that the final gene construct may contain one or more genes of interest, for example, a gene encoding a bioactive metabolic molecule. In addition, cDNA, synthetically produced DNA or chromosomal DNA may be employed utilizing methods and protocols known and practiced by those having skill in the art.

If desired, infectious replication-defective viral vectors may be used to genetically engineer the cells prior to *in vivo* injection of the cells. In this regard, the vectors may be introduced into retroviral producer cells for amphotrophic packaging. The natural expansion of muscle-derived progenitor cells into adjacent regions obviates a large number of injections into or at the site(s) of interest.

In another aspect, the present invention provides *ex vivo* gene delivery to cells and tissues of a recipient mammalian host, including humans, through the use of MDC, e.g., early progenitor muscle cells, that have been virally transduced using an adenoviral vector engineered to contain a heterologous gene encoding a desired gene product. Such an *ex vivo* approach provides the advantage of efficient viral gene transfer, which is superior to direct gene transfer approaches. The *ex vivo* procedure involves the use of the muscle-derived progenitor cells from isolated cells of muscle tissue. The muscle biopsy that will serve as the source of muscle-derived progenitor cells can be obtained from an injury site or from another area that may be more easily obtainable from the clinical surgeon.

It will be appreciated that in accordance with the present invention, clonal isolates can be derived from the population of muscle-derived progenitor cells (*i.e.*, PP6 cells or "slowly adhering" cells using the single plate procedure) using various procedures known in the art, for example, limiting dilution plating in tissue culture medium. Clonal isolates comprise genetically identical cells that originate from a single, solitary cell. In addition, clonal isolates can be derived using FACS analysis as described above, followed by limiting dilution to achieve a single cell per well to establish a clonally isolated cell line. An example of a clonal isolate derived from the PP6 cell population is mc13, which is described in Example 1. Preferably, MDC clonal isolates are utilized in the present methods, as well as for genetic engineering for the expression of one or more bioactive molecules, or in gene replacement therapies.

The MDCs are first infected with engineered viral vectors containing at least one heterologous gene encoding a desired gene product, suspended in a physiologically acceptable carrier or excipient, such as saline or phosphate buffered saline, and then administered to an appropriate site in the host. Consistent with the present invention, the MDCs can be administered to body tissues, including bone, as described above. The desired gene product is expressed by the injected cells, which thus introduce the gene product into the host. The introduced and expressed gene products can thereby be utilized to treat, repair, or ameliorate the injury, dysfunction, or disease, due to their being expressed over long time periods by the MDCs of the invention, having long-term survival in the host.

In animal model studies of myoblast-mediated gene therapy, implantation of 10⁶ myoblasts per 100 mg muscle was required for partial correction of muscle enzyme defects (see, J. E. Morgan *et al.*, 1988, J. Neural. Sci. 86:137; T. A. Partridge *et al.*, 1989, Nature 337:176). Extrapolating from this data, approximately 10¹² MDCs suspended in a physiologically compatible medium can be implanted into muscle tissue for gene therapy for a 70 kg human. This number of MDC of the invention can be produced from a single 100 mg skeletal muscle biopsy from a human source (see below). For the treatment of a specific injury site, an injection of genetically engineered MDC into a given tissue or site of injury comprises a therapeutically effective amount of cells in solution or suspension, preferably, about 10⁵ to 10⁶ cells per cm³ of tissue to be treated, in a physiologically acceptable medium.

### EXAMPLES

### Example 1. MDC Enrichment, Isolation and Analysis According to the Pre-Plating Method.

MDCs were prepared as described (U.S. Pat. No. 6,866,842 of Chancellor et al.). Muscle explants were obtained from the hind limbs of a number of sources, namely from 3-week-old mdx (dystrophic) mice (C57BL/lOScSn mdx/mdx, Jackson Laboratories), 4-6 week-old normal female SD (Sprague Dawley) rats, or SCID (severe combined immunodeficiency) mice. The muscle tissue from each of the animal sources was dissected to remove any bones and minced into a slurry. The slurry was then digested by 1 hour serial incubations with 0.2% type XI collagenase, dispase (grade II, 240 unit), and 0.1 % trypsin at 37 °C. The resulting cell suspension was passed through 18, 20, and 22 gauge needles and centrifuged at 3000 rpm for 5 minutes. Subsequently, cells were suspended in growth medium (DMEM supplemented with 10% fetal bovine serum, 10% horse serum, 0.5% chick embryo extract, and 2% penicillin/streptomycin). Cells were then preplated in collagen-coated flasks (U.S. Pat. No. 6,866,842 of Chancellor et al.). After approximately 1 hour, the supernatant was removed from the flask and re-plated into a fresh collagen-coated flask. The cells which adhered rapidly within this 1 hour incubation were mostly fibroblasts (Z. Qu *et al.*, supra; U.S. Pat. No. 6,866,842 of Chancellor et al.). The supernatant was removed and re-plated after 30-40% of the cells had adhered to each flask. After approximately 5-6 serial platings, the culture was enriched with small, round cells, designated as PP6 cells, which were isolated from the starting cell population and used in further studies. The adherent cells isolated in the early platings were pooled together and designated as PP1-4 cells.

The mdx PP1-4, mdx PP6, normal PP6, and fibroblast cell populations were examined by immunohistochemical analysis for the expression of cell markers. The results of this analysis are shown in Table 1.

**TABLE 1**

| Cell markers expressed in PP1-4 and PP6 cell populations. | | | | |
|---|---|---|---|---|
| | mdx PP1-4 cells | mdx PP6 cells | nor PP6 cells | fibroblasts |
| desmin | +/- | + | + | - |
| CD34 | - | + | + | - |
| Bcl-2 | (-) | + | + | - |
| Flk-1 | na | + | + | - |
| Sca-1 | na | + | + | - |
| M-cadherin | -/+ | -/+ | -/+ | - |
| MyoD | -/+ | +/- | +/- | - |
| myogenin | -/+ | +/- | +/- | - |

Mdx PP1-4, mdx PP6, normal PP6, and fibroblast cells were derived by preplating technique and examined by immunohistochemical analysis. "-" indicates less than 2% of the cells showed expression; "(-)"; "-/+" indicates 5-50% of the cells showed expression; "+/-" indicates ~40-80% of the cells showed expression; "+" indicates that >95% of the cells showed expression; "nor" indicates normal cells; "na" indicates that the immunohistochemical data is not available.

It is noted that both mdx and normal mice showed identical distribution of all the cell markers tested in this assay. Thus, the presence of the mdx mutation does not affect the cell marker expression of the isolated PP6 muscle-cell derived population.

MDCs were grown in proliferation medium containing DMEM (Dulbecco's Modified Eagle Medium) with 10% FBS (fetal bovine serum), 10% HS (horse serum), 0.5% chick embryo extract, and 1% penicillin/streptomycin, or fusion medium containing DMEM supplemented with 2% fetal bovine serum and 1% antibiotic solution. All media supplies were purchased through Gibco Laboratories (Grand Island, N.Y.).

### Example 2. MDC Enrichment, Isolation and Analysis According to the Single Plate Method.

Populations of rapidly- and slowly-adhering MDCs were isolated from skeletal muscle of a mammalian subject. The subject may be a human, rat, dog or other mammal. Biopsy size ranged from 42 to 247 mg.

Skeletal muscle biopsy tissue is immediately placed in cold hypothermic medium (HYPOTHERMOSOL® (BioLife) supplemented with gentamicin sulfate (100 ng/ml, Roche)) and stored at 4°C. After 3 to 7 days, biopsy tissue is removed from storage and production is initiated. Any connective or non-muscle tissue is dissected from the biopsy sample. The remaining muscle tissue that is used for isolation is weighed. The tissue is minced in Hank's Balanced Salt Solution (HBSS), transferred to a conical tube, and centrifuged (2,500×g, 5 minutes). The pellet is then resuspended in a Digestion Enzyme solution (Liberase Blendzyme 4 (0.4-1.0 U/mL, Roche)). 2 mL of Digestion Enzyme solution is used per 100 mg of biopsy tissue and is incubated for 30 minutes at 37 °C on a rotating plate. The sample is then centrifuged (2,500×g, 5 minutes). The pellet is resuspended in culture medium and passed through a 70 µm cell strainer. The culture media used for the procedures described in this Example was Cambrex Endothelial Growth Medium EGM-2 basal medium supplemented with the following components: i. 10% (v/v) fetal bovine serum, and ii. Cambrex EGM-2 SingleQuot Kit, which contains: Insulin Growth Factor-1 (IGF-1), Basic Fibroblast Growth Factor (bFGF), Vascular Endothelial Growth Factor (VEGF), Epidermal Growth Factor (EGF), Hydrocortisone, Heparin, and Ascorbic Acid. The filtered cell solution is then transferred to a T25 culture flask and incubated for 30-120 minutes at 37°C in 5% CO₂. Cells that attach to this flask are the "rapidly-adhering cells".

After incubation, the cell culture supernatant is removed from the T25 flask and placed into a 15 mL conical tube. The T25 culture flask is rinsed with 2 mL of warmed culture medium and transferred to the aforementioned 15 mL conical tube. The 15 mL conical tube is centrifuged (2,500×g, 5 minutes). The pellet is resuspended in culture medium and transferred to a new T25 culture flask. The flask is incubated for ~2 days at 37°C in 5% CO2 (cells that attach to this flask are the "slowly-adhering cells"). After incubation, the cell culture supernatant is aspirated and new culture medium is added to the flask. The flask is then returned to the incubator for expansion. Standard culture passaging is carried out from here on to maintain the cell confluency in the culture flask at less than 50%. Trypsin-EDTA (0.25%, Invitrogen) is used to detach the adherent cells from the flask during passage. Typical expansion of the "slowly-adhering cells" takes an average of 17 days (starting from the day production is initiated) to achieve an average total viable cell number of 37 million cells.

Once the desired cell number is achieved, the cells are harvested from the flask using Trypsin-EDTA and centrifuged (2,500×g, 5 minutes). The pellet is resuspended in BSS-P solution (HBSS supplemented with human serum albumin (2% v/v, Sera Care Life)) and counted. The cell solution is then centrifuged again (2,500×g, 5 minutes), resuspended with Cryopreservation Medium (CryoStor (Biolife) supplemented with human serum albumin (2% v/v, Sera Care Life Sciences)) to the desired cell concentration, and packaged in the appropriate vial for cryogenic storage. The cryovial is placed into a freezing container and placed in the -80°C freezer. Cells are administered by thawing the frozen cell suspension at room temperature with an equal volume of physiologic saline and injected directly (without additional manipulation). The lineage characterization of the slowly adhering cell populations shows: Myogenic (87.4% CD56+, 89.2% desmin+), Endothelial (0.0% CD31+), Hematopoietic (0.3% CD45+), and Fibroblast (6.8% CD90+/CD56-).

Following disassociation of the skeletal muscle biopsy tissue, two fractions of cells were collected based on their rapid or slow adhesion to the culture flasks. The cells were then expanded in culture with growth medium and then frozen in cryopreservation medium (3 × 10⁵ cells in 15 µl) in a 1.5 ml eppendorftube. For the control group, 15 µl of cryopreservation medium alone was placed into the tube. These tubes were stored at -80°C until injection. Immediately prior to injection, a tube was removed from storage, thawed at room temperature, and resuspended with 15 µl of 0.9% sodium chloride solution. The resulting 30 µl solution was then drawn into a 0.5 cc insulin syringe with a 30 gauge needle. The investigator performing the surgery and injection was blinded to the contents of the tubes.

Cell count and viability was measured using a Guava flow cytometer and Viacount assay kit (Guava). CD56 was measured by flow cytometry (Guava) using PE-conjugated anti-CD56 antibody (1:50, BD Pharmingen) and PE-conjugated isotype control monoclonal antibody (1:50, BD Pharmingen). Desmin was measured by flow cytometry (Guava) on paraforrnaldehyde-fixed cells (BD Pharmingen) using a monoclonal desmin antibody (1:100, Dako) and an isotype control monoclonal antibody (1:200, BD Pharmingen). Fluorescent labeling was performed using a Cy3-conjugated anti-mouse IgG antibody (1:250, Sigma). In between steps, the cells were washed with permeabilization buffer (BD Pharmingen). For creatine kinase (CK) assay, 1 × 10⁵ cells were plated per well into a 12 well plate in differentiation-inducing medium. Four to 6 days later, the cells were harvested by trypsinization and centrifuged into a pellet. The cell lysis supernatant was assayed for CK activity using the CK Liqui-UV kit (Stanbio).

### Example 3. Mouse Genetically Modified MDC Treatment of Bone Defects.

### Isolation of muscle derived cells:

MDCs were obtained from mdx mice as described in Example 1.

### Clonal isolation of PP6 muscle-derived progenitor cells:

To isolate clones from the PP6 cell population, PP6 cells were transfected with a plasmid containing the LacZ, mini-dystrophin, and neomycin resistance genes. Briefly, a SmaI/Sa/I fragment containing the neomycin resistance gene from pPGK-NEO was inserted into the SmaI/Sa/I site in pIEPlacZ plasmid containing the LacZ gene, creating the pNEOlacZ plasmid. The XhoI/Sa/I fragment from DysM3 which contains the short version of the dystrophin gene (K. Yuasa et al., 1998, FEBS Left. 425:329 336; gift from Dr. Takeda, Japan) was inserted into Sa/I site in the pNEOlacZ to generate a plasmid which contains the mini-dystrophin, LacZ, and neomycin resistance genes. The plasmid was linearized by Sa/I digestion prior to transfection.

PP6 cells were transfected with 10 µg of the linear plasmid containing mini-dystrophin, LacZ, and neomycin resistance gene using the LIPOFECTAMINE^{™} Reagent (Gibco BRL) according to the manufacturer's instructions. At 72 hours after transfection, cells were selected with 3000 µg/ml of G418 (Gibco BRL) for 10 days until discrete colonies appeared. Colonies were then isolated and expanded to obtain a large quantity of the transfected cells, and then tested for expression of LacZ. One of these PP6-derived clones, mc13, was used for further study.

### Immunohistochemistry:

PP6, mc13, and mouse fibroblast cells were plated in a 6-well culture dish and fixed with cold methanol for 1 minute. Cells were then washed with phosphate buffered saline (PBS), and blocked with 5% horse serum at room temperature for 1 hour. The primary antibodies were diluted in PBS as follows: anti-desmin (1:100, Sigma), biotinylated anti-mouse CD34 (1:200, Pharmingen), rabbit anti-mouse Bcl-2 (1:500, Pharmingen), rabbit anti-mouse M-cadherin (1:50, gift from Dr. A. Wemig), mouse anti-mouse MyoD (1:100, Pharmingen), mouse anti-rat myogenin (1:100, Pharmingen), rabbit anti-mouse Flk-1 (1:50, Research Diagnostics), and biotinylated Sca-1 (1:100, Pharmingen). Cells were incubated with the primary antibodies at room temperature overnight. Cells were then washed and incubated with the appropriate biotinylated secondary antibodies for 1 hour at room temperature. Subsequently, the cells were rinsed with PBS then incubated at room temperature with 1/300 streptavidin conjugated with Cy3 fluorochrome for 1 hour. Cells were then analyzed by fluorescence microscopy. For each marker, the percentage of stained cells was calculated for 10 randomly chosen fields of cells.

Cryosections of muscle samples from a four week old normal mouse (C-57 BL/6J, Jackson Laboratories) were fixed with cold acetone for 2 minutes and pre-incubated in 5% horse serum diluted in PBS for 1 hour. For CD34, Bcl-2, and collagen type IV, the following primary antibodies were used: biotin anti-mouse CD34 (1:200 in PBS, Pharmingen), rabbit anti-mouse Bcl-2 (1:1000, Pharmingen), and rabbit anti-mouse collagen type IV (1:100 in PBS, Chemicon). For dystrophin staining, sheep-anti-human DY10 antibody (1:250 dilution in PBS) was used as the primary antibody, and the signal was amplified using anti-sheep-biotin (1:250 dilution in PBS), and streptavidin-FITC (1:250 dilution in PBS).

### Stimulation with rhBMP-2, osteocalcin staining, and alkaline phosphatase assay:

Cells were plated in triplicate at a density of 1-2x10⁴ cells per well in 12 well collagen-coated flasks. The cells were stimulated by the addition of 200 ng/ml recombinant human BMP-2 (rhBMP-2) to the growth medium. The growth medium was changed on days 1, 3, and 5 following the initial plating. A control group of cells was grown in parallel without added rhBMP-2. After 6 days with or without rhBMP-2 stimulation, cells were counted using a microcytometer and analyzed for osteocalcin and alkaline phosphatase expression. For osteocalcin staining, cells were incubated with goat anti-mouse osteocalcin antibodies (1:100 in PBS, Chemicon), followed by incubation with anti-goat antibodies conjugated with the Cy3 fluorochrome. To measure alkaline phosphatase activity, cell lysates were prepared and analyzed using a commercially available kit that utilizes color change in the reagent due to the hydrolysis of inorganic phosphate from p-nitrophenyl phosphate (Sigma). The resulting color change was measured on a spectrophotometer, and the data were expressed as international units ALP activity per liter normalized to 106 cells. Statistical significance was analyzed using student's t-test (p<0.05).

### In vivo differentiation of mc13 cells in myogenic and osteogenic lineage--Myogenic:

The mc13 cells (5x10⁵ cells) were injected intramuscularly in the hind limb muscle of mdx mice. The animals were sacrificed at 15 days post-injection, and the injected muscle tissue was frozen, cryostat sectioned, and assayed for dystrophin (see above) and LacZ expression. To test for LacZ expression, the muscle sections were fixed with 1% glutaraldehyde and then were incubated with X-gal substrate (0.4 mg/ml 5-bromochloro-3 indolyl-β-D-galactoside (Boehringer-Mannheim), 1 mM MgCl₂, 5 mM K₄Fe(CN)₆, and 5 mM K₃Fe(CN)₆ in phosphate buffered saline) for 1-3 hours. Sections were counter-stained with eosin prior to analysis. In parallel experiments, mc13 cells (5x10⁵ cells) were injected intravenously in the tail vein of mdx mice. The animals were sacrificed at 7 days post-injection and hind limbs were isolated and assayed for the presence of dystrophin and β-galactosidase as described.

### Osteogenic:

To construct the adenovirus BMP-2 plasmid (adBMP-2), the rhBMP-2 coding sequence was excised from the BMP-2-125 plasmid (Genetics Institute, Cambridge, Mass.) and subcloned into a replication defective (E1 and E3 gene deleted) adenoviral vector containing the HuCMV promoter. Briefly, the BMP-2-125 plasmid was digested with Sa/I, resulting in a 1237 base pair fragment containing the rhBMP-2 cDNA. The rhBMP-2 cDNA was then inserted into the Sa/I site of the pAd.lox plasmid, which placed the gene under the control of the HuCMV promoter. Recombinant adenovirus was obtained by co-transfection of pAd.lox with psi-5 viral DNA into CREW cells. The resulting adBMP-2 plasmid was stored at -80 °C until further use.

Mc 13 cells were trypsinized and counted using a microcytometer prior to infection. Cells were washed several times using HBSS (GibcoBRL). Adenovirus particles equivalent to 50 multiplicity of infection units were premixed into HBSS then layered onto the cells. Cells were incubation at 37 °C for 4 hours, and then incubated with an equal volume of growth medium. Injections of 0.5-1.0x10⁶ cells were performed using a 30-gauge needle on a gas-tight syringe into exposed triceps surae of SCID mice (Jackson Laboratories). At 14-15 days, the animals were anesthetized with methoxyflurane and sacrificed by cervical dislocation. The hind limbs were analyzed by radiography. Subsequently, the triceps surae were isolated and flash frozen in 2-methylbutane buffered in phosphate buffered saline, and pre-cooled in liquid nitrogen. The frozen samples were cut into 5-10 µm sections using a cryostat (Microm, HM 505 E, Fisher Scientific) and stored at -20 °C for further analysis.

RT-PCR analysis: Total RNA was isolated using TRIZOL® reagent (Life Technologies). Reverse transcription was carried out using SUPERSCRIPT^{™} Preamplification System for First Strand cDNA Synthesis (Life Technologies) according to the instructions of the manufacturer. Briefly, 100 ng random hexamers were annealed to 1 µg total RNA at 70 °C for 10 minutes, and then chilled on ice. Reverse transcription was carried out with 2 µl 10x PCR buffer, 2 µl 25 mM MgCl₂, 1 µl 10 mM dNTP mix, 2 µl 0.1 M DTT, and 200 U superscript 11 reverse transcriptase. The reaction mixture was incubated for 50 minutes at 42 °C.

Polymerase chain reaction (PCR) amplification of the targets was performed in 50 µl reaction mixture containing 2 µl of reverse transcriptase reaction product, 100 µl (5 U) Taq DNA polymerase (Life Technologies), and 1.5 mM MgCl₂. The CD34 PCR primers were designed using Oligo software and had the following sequences: CD34 UP: TAA CTT GAC TTC TGC TAC CA (SEQ ID NO:1); and CD34 DOWN: GTG GTC TTA CTG CTG TCC TG (SEQ ID NO:2). The other primers were designed according to previous studies (J. Rohwedel et al., 1995, Exp. Cell Res. 220:92 100; D. D. Comelison et al., 1997, Dev. Biol. 191:270 283), and had the following sequences: C-MET UP: GAA TGT CGT CCT ACA CGG CC (SEQ ID NO:3); C-MET DOWN: CAC TAC ACA GTC AGG ACA CTG C (SEQ ID NO:4); MNF UP: TAC TTC ATC AAA GTC CCT CGG TC (SEQ ID NO:5); MNF DOWN: GTA CTC TGG AAC AGA GGC TAA CTT (SEQ ID NO:6); BCL-2 UP: AGC CCT GTG CCA CCA TGT GTC (SEQ ID NO:7); BCL-2 DOWN: GGC AGG TTT GTC GAC CTC ACT (SEQ ID NO:8); MYOGENIN UP: CAA CCA GGA GGA GCG CGA TCT CCG (SEQ ID NO:9); MYOGENIN DOWN: AGG CGC TGT GGG AGT TGC ATT CAC T (SEQ ID NO: 10); MYOD UP: GCT CTG ATG GCA TGA TGG ATT ACA GCG (SEQ ID NO:11); and MYOD DOWN: ATG CTG GAC AGG CAG TCG AGG C (SEQ ID NO:12).

The following PCR parameters were used: 1) 94 °C for 45 seconds; 2) 50 °C for 60 seconds (CD34) or 60 °C for 60 seconds (for myogenin and c-met); and 3) 72 °C for 90 seconds for 40 cycles. PCR products were checked by agarose-TBE-ethidium bromide gels. The sizes of the expected PCR products are: 147 bp for CD34; 86 bp for myogenin; and 370 bp for c-met. To exclude the possibility of genomic DNA contamination, two control reactions were completed: 1) parallel reverse transcription in the absence of reverse transcriptase, and 2) amplification of β-actin using an intron-spanning primer set (Clonetech).

### Skull defect assay:

Three 6-8 week old female SCID mice (Jackson Laboratories) were used in control and experimental groups. The animals were anesthetized with methoxyflurane and placed prone on the operating table. Using a number 10 blade, the scalp was dissected to expose the skull, and the periosteum was stripped. An approximately 5 mm full-thickness circular skull defect was created using a dental burr, with minimal penetration of the dura. A collagen sponge matrix (HELISTAT^{™}, Colla-T c, Inc.) was seeded with 0.5-1.0x10⁶ MDC either with or without adBMP-2 transduction, and placed into the skull defect. The scalp was closed using a 4-0 nylon suture, and the animals were allowed food and activity. After 14 days, the animals were sacrificed, and the skull specimens were observed and then analyzed microscopically. For von Kossa staining, skull specimens were fixed in 4% formaldehyde and then soaked in 0.1 M AgNO₃ solution for 15 minutes. The specimens were exposed to light for at least 15 minutes, washed with PBS, and then stained with hematoxylin and eosin for viewing.

### Fluorescence in situ hybridization using Y-probes:

The cryosections were fixed for 10 minutes in 3:1 methanol/glacial acetic acid (v:v) and air dried. The sections were then denatured in 70% formamide in 2xSSC (0.3 M NaCl, 0.03 M NaCitrate) pH 7.0 at 70 °C for 2 minutes. Subsequently, the slides were dehydrated with a series of ethanol washes (70%, 80%, and 95%) for 2 minutes at each concentration. The Y-chromosome specific probe (Y. Fan et al., 1996, Muscle Nerve 19:853 860) was biotinylated using a BioNick kit (Gibco BRL) according to the manufacturer's instructions. The biotinylated probe was then purified using a G-50 Quick Spin Column (Boehringer-Mannheim), and the purified probe was lyophilized along with 5 ng/ml of sonicated herring sperm DNA. Prior to hybridization, the probe was resuspended in a solution containing 50% formamide, 1xSSC, and 10% dextran sulfate. After denaturation at 75 °C for 10 minutes, the probe was placed on the denatured sections and allowed to hybridize overnight at 37 °C. After hybridization, the sections were rinsed with 2xSSC solution pH 7.0 at 72 °C for 5 minutes. The sections were then rinsed in BMS solution (0.1 M NaHCO₃, 0.5 M NaCl, 0.5% NP-40, pH 8.0). The hybridized probe was detected with fluorescein labeled avidin (ONCOR, Inc). The nuclei were counter-stained with 10 ng/ml ethidium bromide in VECTASHIELD® mounting medium (Vector, Inc).

### Marker analysis of mc13 cells:

The biochemical markers expressed by mc13, PP6, and fibroblast cells were analyzed using RT-PCR and immunohistochemistry. Table 2 (below) shows that mc13 cells expressed Flk-1, a mouse homologue of the human KDR gene, which was recently identified as a marker of hematopoietic cells with stem cell-like characteristics (B. L. Ziegler *et al., supra*), but did not express CD34 or CD45. However, other clonal isolates derived from the PP6 MDC of the present invention expressed CD34, as well as other PP6 cell markers. It will be appreciated by those skilled in the art that the procedures described herein can be used to clone out the PP6 muscle-derived progenitor cell population and obtain clonal isolates that express cell markers characteristic of the muscle-derived progenitor cells. Such clonal isolates can be used in accordance with the methods of the invention. For example, the clonal isolates express progenitor cell markers, including desmin, CD34, and Bcl-2. Preferably, the clonal isolates also express the Sca-1 and Flk-1 cell markers, but do not express the CD45 or c-Kit cell markers.

**TABLE 2**

| Cellmarkers expressed by mdx PP6, mdx mc13, and fibroblast cells. | | | | | | |
|---|---|---|---|---|---|---|
| | PP6 cells | | MC13 cells | | Fibroblasts | |
| | imm | RT-PCR | imm | RT-PCR | imm | RT-PCR |
| desmin | + | na | + | na | - | na |
| CD34 | + | + | - | - | - | - |
| Bcl-2 | + | na | +/- | na | - | na |
| Flk-1 | + | na | + | na | - | na |
| Sca-1 | + | na | + | na | - | na |
| M-cadherin | -/+ | na | + | na | - | na |
| Myogenin | +/- | + | +/- | + | - | - |
| c-met | na | + | na | + | na | - |
| MNF | na | + | na | + | na | - |
| MyoD | -/+ | + | na | + | na | - |
| c-Kit | - | na | - | na | na | na |
| CD45 | - | na | - | na | na | na |

Cells were isolated as described above and examined by immunohistochemical analysis. "-" indicates that 0% of the cells showed expression; "+" indicates that >98% of the cells showed expression; "+/-" indicates that 40-80% of the cells showed expression; "-/+" indicates that 5-30% of the cells showed expression; and "na" indicates that the data is not available.

### In vivo localization of CD34⁺ and Bcl-2⁺ cells:

To identify the location of CD34⁺ and Bcl-2⁺ cells *in vivo,* muscle tissue sections from the triceps surae of normal mice were stained using anti-CD34 and anti-Bcl-2 antibodies. The CD34 positive cells constituted a small population of muscle derived cells (Figure 1 A) that were also positive for desmin (Figure 1B). Co-staining the CD34+, desmin+ cells with anti-collagen type IV antibody localized them within the basal lamina (Figures 1B and 1D). As indicated by the arrowheads in Figures 1A-D, small blood vessels were also positive for CD34 and collagen type IV, but did not co-localize with the nuclear staining. The expression of CD34 by vascular endothelial cells has been shown in previous studies (L. Fina *et al., supra*). The Bcl-2+, desmin+ cells were similarly identified (Figures 1E-1H) and localized within the basal lamina (Figures 1F and 1H). The sections were also stained for M-cadherin to identify the location of satellite cells (Figure 1I). The satellite cells were identified at similar locations as CD34+, desmin+, or Bcl-2+, desmin+cells (arrow, Figure 1I). However, multiple attempts to co-localize CD34 or Bcl-2 with M-cadherin were unsuccessful, suggesting that M-cadherin expressing cells do not co-express either Bcl-2 or CD34. This is consistent with PP6 cells expressing high levels of CD34 and Bcl-2, but expressing minimal levels of M-cadherin, as disclosed herein.

### In vitro differentiation of clonal muscle progenitor cells into osteogenic lineage:

Mcl3 cells were assessed for osteogenic differentiation potential by stimulation with rhBMP-2. Cells were plated on 6-well culture dishes and grown to confluency in the presence or absence of 200 ng/ml rhBMP-2. Within 34 days, mc13 cells exposed to rhBMP-2 showed dramatic morphogenic changes compared to cells without rhBMP-2. In the absence of rhBMP-2, mc13 cells began to fuse into multinucleated myotubes (Figure 2A). When exposed to 200 ng/ml rhBMP-2, however, cells remained mononucleated and did not fuse (Figure 2B). When cell density reached >90% confluency, the untreated culture fused to form multiple myotubes (Figure 2C), while the treated cells became circular and hypertrophic (Figure 2D). Using immunohistochemistry, these hypertrophic cells were analyzed for the expression of osteocalcin. Osteocalcin is a matrix protein that is deposited on bone, specifically expressed by osteoblasts. In contrast to the untreated group, the rhBMP-2 treated hypertrophic cells showed significant expression of osteocalcin (Figure 2E), thus suggesting that mc13 cells are capable of differentiating into osteoblasts upon exposure to rhBMP-2.

Mc13 cells were then analyzed for expression of desmin following rhBMP-2 stimulation. Newly isolated mc13 cells showed uniform desmin staining (Figures 3A and 3B). Within 6 days of exposure to rhBMP-2, only 30-40% of mc13 cells showed desmin staining. In the absence of rhBMP-2 stimulation, approximately 90-100% of mc13 cells showed desmin staining (Figure 3C). This result suggests that stimulation of mc13 cells with rhBMP-2 results in the loss of myogenic potential for these cells.

In addition, mc13 cells were analyzed for the expression of alkaline phosphatase following rhBMP-2 stimulation. Alkaline phosphatase has been used as a biochemical marker for osteoblastic differentiation (T. Katagiri et al., 1994, J. Cell Biol. 127:1755 1766). As shown in Figure 3D, alkaline phosphatase expression of mc13 cells was increased more than 600 fold in response to rhBMP-2. PP1 4 cells, used as a control, did not show increased alkaline phosphatase activity in response to rhBMP-2 (Figure 3D). Taken together, these data demonstrate that cells of a PP6 clonal isolate, *e.g.*, mc13 cells, can lose their myogenic markers and differentiate through the osteogenic lineage in response to rhBMP-2 exposure *in vitro.*

### In vivo differentiation of mc13 cells into myogenic and osteogenic lineages:

To determine whether mc13 cells were capable of differentiating through the myogenic lineage *in vivo,* the cells were injected into the hind limb muscle tissue of mdx mice. The animals were sacrificed 15 days following injection, and their hind limbs were harvested for histological and immunohistochemical analysis. Several myofibers showed LacZ and dystrophin staining in the region surrounding the injection site (Figures 4A and 4B), indicating that mc13 cells can differentiate through the myogenic lineage *in vivo* and enhance muscle regeneration and restore dystrophin in the dystrophic muscle.

In a parallel experiment, mc13 cells were injected intravenously into the tail vein of mdx mice. The animals were sacrificed at 7 days post-injection, and the hind limb muscles were harvested for histological and immunohistochemical analysis. Several hind limb muscle cells showed LacZ and dystrophin staining (Figures 4C and 4D; see also "*"), suggesting that mc13 cells can be delivered systemically to the target tissue for rescue of dystrophin expression.

To test the pluripotent characteristics of mc13 cells *in vivo,* the cells were transduced with an adenoviral vector encoding rhBMP-2 (adBMP-2). The mc13 cells with adBMP-2 were then injected into hind limbs of SCID mice. The animals were sacrificed at 14 days post-injection, and the hind limbs were removed for histochemical and immunochemical analysis. Enzyme-linked immunosorbent assay (ELISA) analysis of mc13 cells transduced with adBMP-2 showed that infected cells were capable of producing rhBMP-2. Radiographic analysis of hind limbs of injected SCID mice revealed robust ectopic bone formation within 14 days of injection (Figure 4E). Histological analysis using LacZ staining of the ectopic bone shows that LacZ positive mc13 cells were uniformly located within the mineralized matrix or lacunae, a typical location where osteoblasts and osteocytes are found (Figure 4F).

To further confirm the role of mc13 in formation of the ectopic bone, the muscle sections were also stained for presence of dystrophin. As shown in Figure 4G, the ectopic bone contained cells highly positive for dystrophin, suggesting that mc13 cells are intimately participating in bone formation. As a control, similar experiments were carried out with fibroblasts. Fibroblasts were found to support robust ectopic bone formation, but the injected cells were uniformly found outside of the bone, and none could be located within the mineralized matrix. This suggests that the fibroblasts are capable of delivering rhBMP-2 to form ectopic bone, but are unable to differentiate into osteoblasts. In this case, the cells participating in mineralization of the ectopic bone are most likely derived from the host tissue. Thus, these results demonstrate that mc 13 cells can differentiate into osteoblasts, both *in vivo* and *in vitro,*

### Enhancement of bone healing by genetically engineered muscle-derived cells:

Skull defects (approximately 5 mm) were created in skeletally mature (6-8 weeks old) female SCID mice using a dental burr as described above. Previous experiments have demonstrated that 5 mm skull defects are "non-healing" (P. H. Krebsbach et al., 1998, Transplantation 66:1272-1278). The skull defect was filled with a collagen sponge matrix seeded with mc13 cells transduced or not transduced with adBMP-2. These mice were sacrificed at 14 days, and the healing of the skull defect was analyzed. As shown in Figure 5A, the control group treated with mc13 cells without rhBMP-2 showed no evidence of healing of the defect. In contrast, the experimental group treated with mc13 cells transduced to express rhBMP-2 showed almost a full closure of the skull defect at 2 weeks (Figure 5B). The von Kossa staining, which highlights mineralized bone, showed robust bone formation in the group treated with mc13 cells transduced to express rhBMP-2 (Figure 5D), but minimal bone formation was observed in the control group (Figure 5C).

The area of new bone in the experimental group was analyzed by fluorescence *in situ* hybridization (FISH) with a Y-chromosome specific probe to identify transplanted cells. As shown in Figure 5E, Y-chromosome positive cells were identified within the newly formed bone, indicating active participation of transplanted cells in bone formation under the influence of rhBMP-2. The Y-chromosome negative cells were also identified within the newly formed skull, thus indicating active participation of host-derived cells as well. These results demonstrate that mc13 cells can mediate healing of a "non-healing" bone defect upon stimulation with rhBMP-2, and indicate that the MDC of the present invention can be used in the treatment of bone defects, injuries, or traumas.

### Example 4. Increase of Bone Density and Bone Volume in Human Tissue through

### Administration of MDCs.

In this study, a 3-dimensional (3D) culture system involving cell pellets, commonly used to induce progenitor cells to undergo chondrogenesis, (Yoo et al., JBJS, 1998, 80(12):1745-1757) was used to evaluate the ability of hMDCs to undergo mineralization. Using micro-computed tomography (µCT) analysis, we were able to observe the same pellet over time and determine the rate of mineralization for each cell population tested. T 5 he data below show that all hMDCs in this study were capable of mineralization, with most doing so by Day 7 of culture. Also, hMDCs increased their expression of Collagen type I (ColI), the main collagen found in bone, suggesting osteogenic differentiation. Unlike murine muscle cells, hMDCs did not require BMP stimulation to undergo mineralization, and were positive for alkaline phosphatase prior to osteogenic stimulation. Cells varied in CD56 expression between donors (CD56+ range for the 4 female populations = 42%- 82% and the 4 male populations = 55%-90%). Moreover, this osteogenic assay showed that hMDCs with low CD56 expression did not mineralize as quickly as those expressing higher levels, showing that CD56 may be a marker for the osteogenic potential of hMDCs.

In small skeletal muscle biopsies taken from 4 human females (ages 22, 24, 24, 25) and 4 human males (ages 20, 26, 28, 30), muscle derived cell (MDC) populations ("slowly adhering cells") were collected from the late preplates according to the single plate method described in Example 2, above. Prior to stimulation, cells were cultured as described in Example 2. The cells were then induced as pellets (250,000 cells/pellet) in osteogenic medium (OSM) (DMEM supplemented with 10% fetal bovine serum, 1% penicillin/streptomycin, 10⁻⁷ M dexamethasone, 50 µg/mL ascorbic-acid-2-phosphate and 10⁻² M β-glycerophosphate) (n=6 pellets per population) for 28 days. The bone volume and density of the pellets were measured by µCT analysis at 7, 14, 21 and 28 days for bone volume (BV) and bone density (BD). Gene expression for collagen type I (ColI) was determined by quantitative RT-PCR on RNA isolated on the day the pellets were made (Day 0) and after 28 days in OSM (Day 28). Statistical analysis was performed using a Two-way ANOVA for BV and BD and a t-test for gene expression between days 0 and 28 within each sex. P-values < 0.05 were considered significant. This data is represented in Figures 6A, 6B, 7A, and 7B showing the mean ± SEM (n=6 populations/sex).

All hMDCs formed pellets, and calcification was evident in most populations as early as 7 days. The female and male cell population with the lowest percentage of CD56 began displaying calcified tissue only after 14 days in culture. The mean BV and BD over time in all cell populations tested are represented in Figures 6A and 6B, respectively. A significant increase in BV was observed between 7 and 28 days in the male hMDCs. In the case of the female hMDCs, the BV of the pellets increased significantly between days 14 and 21. The BD in both female and male hMDCs populations progressed every 7 days. Pellets scanned at Days 21 and 28 had denser mineralization than pellets scanned at Days 7 and 14. No sex-related differences were observed at all time points tested for both BV and BD (Figures 6A and 6B). These findings suggest that hMDCs are capable of producing mineralized bone tissue.

Collagen type I (ColI), which is an osteoblast gene marker and is the collagen found in bone, was measured to determine whether hMDCs differentiated into osteoblasts when using the osteogenic pellet culture system. ColI gene expression was significantly increased in both male and female hMDCs after 28 days of culture in OSM (Figure 7). Thus, this data shows gene expression consistent with MDCs that may have differentiated into osteoblasts..

## Claims

1. A cell population containing muscle derived cells (MDCs) for use in treating a bone defect, disease or pathology, or for augmenting bone mass or density in a mammalian subject, wherein said cell population is obtained by a method comprising:
(a) suspending cells isolated from mammalian skeletal muscle in a first cell culture container for between 30 and 120 minutes which is sufficient to adhere a first cell population to the container and to leave a second cell population remaining unadhered and in a culture medium in the container;
(b) transferring the culture medium and second cell population from the first cell culture container to a second cell culture container;
(c) allowing cells from the second cell population to attach to the second cell culture container; and
(d) isolating the cells attached to the second cell culture container to obtain said cell population containing MDCs.

2. A cell population for use as claimed in claim 1 comprising:
(1) cooling the cells isolated from mammalian skeletal muscle to a temperature lower than 10° C and storing the cells for 1 -7 days;
(2) suspending said mammalian skeletal muscle cells in said first cell culture container between 30 and 120 minutes;
(3) decanting the media from the first cell culture container to the second cell culture container;
(4) isolating the MDCs from the walls of the second cell culture container;
(5) culturing MDCs to expand their number; and thereafter
(6) freezing the MDCs to a temperature below -30° C.

3. The cell population for use of claim 2, wherein the skeletal muscle cells are human.

4. The cell population for use of claim 3, wherein the human skeletal muscle cells have been isolated from a human subject before a bone defect, disease or pathology has begun in the human subject.

5. The cell population for use of claim 3, wherein the human skeletal muscle cells have been isolated from a human subject alter a bone defect, disease or pathology has begun in the human subject.

6. The cell population for use according to any preceding claim wherein the bone disease, disorder or defect is selected from osteoporosis, Paget's disease, osteogenesis imperfecta, bone fracture, osteomalacia, decrease in bone trabecular strength, decrease in bone cortical strength, and decrease in bone density with old age.

7. The cell population for use according to any preceding claim wherein said cell population increases bone mass in said mammalian subject.

## Patentansprüche

1. Zellpopulation, die von Muskeln stammende Zellen (MDCs, muscle derived cells) enthält, zur Verwendung bei der Behandlung eines Knochenschadens, einer Knochenkrankheit oder eines pathologischen Knochenbefunds, oder zur Steigerung der Knochenmasse oder Knochendichte bei einem Säugetier-Individuum, wobei die Zellpopulation erhalten wird durch ein Verfahren, das aufweist:
(a) Suspendieren von Zellen, die aus einem Säugetier-Skelettmuskel isoliert wurden, in einem ersten Zellkultur-Behälter für zwischen 30 und 120 Minuten, was ausreichend ist, um eine erste Zellpopulation an dem Behälter zu befestigen und eine zweite Zellpopulation nicht befestigt und in einem Kulturmedium in dem Behälter zu lassen;
(b) Überführen des Kulturmediums und der zweiten Zellpopulation aus dem ersten Zellkultur-Behälter in einen zweiten Zellkultur-Behälter;
(c) Zulassen, dass Zellen aus der zweiten Zellpopulation an dem zweiten Zellkultur-Behälter anhaften; und
(d) Isolieren der an dem zweiten Zellkultur-Behälter haftenden Zellen, um die MDCs enthaltende Zellpopulation zu erhalten.

2. Zellpopulation zur Verwendung wie in Anspruch 1 beansprucht, aufweisend:
(1) Abkühlen der aus einem Säugetier-Skelettmuskel isolierten Zellen auf eine Temperatur, die niedriger ist als 10 °C, und Aufbewahren der Zellen für ein bis sieben Tage;
(2) Suspendieren der Säugetier-Skelettmuskelzellen in dem ersten Zellkultur-Behälter für zwischen 30 und 120 Minuten;
(3) Dekantieren der Medien aus dem ersten Zellkultur-Behälter in den zweiten Zellkultur-Behälter;
(4) Isolieren der MDCs von den Wänden des zweiten Zellkultur-Behälters;
(5) Kultivieren der MDCs, um ihre Anzahl zu vergrößern; und danach
(6) Tiefkühlen der MDCs auf eine Temperatur unter 30 °C.

3. Zellpopulation zur Verwendung nach Anspruch 2, wobei die Skelettmuskelzellen menschlich sind.

4. Zellpopulation zur Verwendung nach Anspruch 3, wobei die menschlichen Skelettmuskelzellen aus einem menschlichen Individuum isoliert wurden bevor ein Knochenschaden, eine Knochenkrankheit oder ein pathologischer Knochenbefund bei dem menschlichen Individuum begonnen hat.

5. Zellpopulation zur Verwendung nach Anspruch 3, wobei die menschlichen Skelettmuskelzellen aus einem menschlichen Individuum isoliert wurden nachdem ein Knochenschaden, eine Knochenkrankheit oder ein pathologischer Knochenbefund bei dem menschlichen Individuum begonnen hat.

6. Zellpopulation zur Verwendung nach einem vorangehenden Anspruch, wobei die Knochenkrankheit, die Knochenstörung oder der Knochenschaden ausgewählt ist aus Osteoporose, Paget-Krankheit, Osteogenesis imperfecta, Knochenbruch, Osteomalazie, Abnahme der trabekulären Knochenfestigkeit, Abnahme der kortikalen Knochenfestigkeit, und Abnahme der Knochendichte im hohen Alter.

7. Zellpopulation zur Verwendung nach einem vorangehenden Anspruch, wobei die Zellpopulation die Knochenmasse bei dem Säugetier-Individuum erhöht.

## Revendications

1. Population de cellules contenant des cellules d'origine musculaire (MDC) pour utilisation dans le traitement d'un défaut, d'une maladie ou d'une pathologie des os, ou pour augmenter la masse ou la densité osseuse chez un sujet mammifère, ladite population de cellules étant obtenue par un procédé comprenant :
(a) la mise en suspension des cellules isolées à partir de muscle squelettique de mammifère, dans un premier récipient de culture cellulaire pendant 30 à 120 minutes, ce qui suffit pour faire adhérer une première population de cellules au récipient et pour laisser une deuxième population de cellules qui reste non adhérée et dans un milieu de culture dans le récipient ;
(b) le transfert du milieu de culture et la deuxième population de cellules depuis le premier récipient de culture cellulaire dans un deuxième récipient de culture cellulaire ;
(c) l'opération consistant à laisser les cellules de la deuxième population de cellules s'attacher au deuxième récipient de culture cellulaire ; et
(d) l'isolation des cellules attachées au deuxième récipient de culture cellulaire afin d'obtenir ladite population de cellules contenant des MDC.

2. Population de cellules pour utilisation selon la revendication 1 comprenant :
(1) le refroidissement des cellules isolées à partir de muscle squelettique de mammifère à une température inférieure à 10°C et le stockage des cellules pendant 1 à 7 jours ;
(2) la mise en suspension desdites cellules de muscle squelettique de mammifère dans ledit premier récipient de culture cellulaire pendant 30 à 120 minutes ;
(3) la décantation du milieu du premier récipient de culture cellulaire dans le deuxième récipient de culture cellulaire ;
(4) l'isolation des MDC à partir des parois du deuxième récipient de culture cellulaire ;
(5) la culture des MDC pour augmenter leur nombre ; et ensuite
(6) la congélation des MDC à une température inférieure à -30°C.

3. Population de cellules pour utilisation selon la revendication 2, dans laquelle les cellules de muscle squelettique sont humaines.

4. Population de cellules pour utilisation selon la in 3, dans laquelle les cellules de muscle squelettique humaines ont été isolées à partir d'un sujet humain avant qu'un défaut, une maladie ou une pathologie des os ait commencé chez le sujet humain.

5. Population de cellules pour utilisation selon la revendication 3, dans laquelle les cellules de muscle squelettique humaines ont été isolées à partir d'un sujet humain après qu'un défaut, une maladie ou une pathologie des os a commencé chez le sujet humain.

6. Population de cellules pour utilisation selon l'une quelconque des revendications précédentes, dans laquelle la maladie, le trouble ou le défaut des os est choisi parmi l'ostéoporose, la maladie de Paget, l'ostéogenése imparfaite, une fracture osseuse, 1'ostéomalacie, une diminution de la résistance trabéculaire osseuse, une diminution de la résistance corticale osseuse, et une diminution de la densité osseuse du fait d'un âge avancé.

7. Population de cellules pour utilisation selon l'une quelconque des revendications précédentes, laquelle population de cellules augmente la masse osseuse dans ledit sujet mammifère.
